# EUROPEAN PATENT SPECIFICATION

(11) **EP 3 507 300 B1**
(45) Date of publication and mention of the grant of the patent: **28.12.2022**
(21) Application number: 17844710.8
(22) Date of filing: 04.09.2017
(51) Int. Cl.: C07K 14/475, C07K 14/435, A61K 38/00, A61K 38/18, A61P 17/02, A61P 43/00

(54) **WOUND HEALING PEPTIDE**
WUNDHEILENDES PEPTID
PEPTIDE DE CICATRISATION DE PLAIE

(30) Priority: 02.09.2016 AU 2016903523; 22.12.2016 AU 2016905327
(43) Date of publication of application: 10.07.2019
(73) Proprietor: James Cook University, Townsville, Queensland 4811 (AU)
(72) Inventor: LOUKAS, Alex, Cairns, Queensland 4878 (AU); SMOUT, Michael, Smithfield, Queensland 4878 (AU); DALY, Norelle, Trinity Beach, Queensland 4879 (AU)
(74) Representative: IPrime Rentsch Kaelin AG
(86) International application number: PCT/AU2017/050959
(87) International publication number: WO 2018/039748

(56) References cited:
- WO-A1-93/15195
- US-B1- 7 427 595
- MICHAEL J. SMOUT ET AL: "A Granulin-Like Growth Factor Secreted by the Carcinogenic Liver Fluke, Opisthorchis viverrini, Promotes Proliferation of Host Cells", PLOS PATHOGENS, vol. 5, no. 10, 9 October 2009 (2009-10-09), pages 1-16, XP055192406, DOI: 10.1371/journal.ppat.1000611
- MICHAEL J. SMOUT ET AL: "Carcinogenic Parasite Secretes Growth Factor That Accelerates Wound Healing and Potentially Promotes Neoplasia", PLOS PATHOGENS, vol. 11, no. 10, 20 October 2015 (2015-10-20), pages 1-20, XP055469897, DOI: 10.1371/journal.ppat.1005209
- DATABASE UniProt [Online] 25 January 2012 (2012-01-25), "SubName: Full=Granulin-like protein {ECO:0000313|EMBL:RJW65418.1}; SubName: Full=Granulins {ECO:0000313|EMBL:GAA49649.1};", XP002797809, retrieved from EBI accession no. UNIPROT:G7Y9L4 Database accession no. G7Y9L4
- W.F. VRANKEN ET AL: "A 30-residue fragment of the carp granulin-1 protein folds into a stack of two beta-hairpins similar to that found in the native protein", JOURNAL OF PEPTIDE RESEARCH, vol. 53, no. 5, 1 May 1999 (1999-05-01), pages 590-597, XP55669874, OXFORD; GB ISSN: 1397-002X, DOI: 10.1034/j.1399-3011.1999.00048.x
- MICHAEL J SMOUT ET AL: "Expression, refolding and purification of-GRN-1, a granulin-like growth factor from the carcinogenic liver fluke, that causes proliferation of mammalian host cells", PROTEIN EXPRESSION AND PURIFICATION, vol. 79, no. 2, 2 July 2011 (2011-07-02), pages 263-270, XP028258817, ISSN: 1046-5928, DOI: 10.1016/J.PEP.2011.06.018 [retrieved on 2011-07-02]
- PARAMJIT S. BANSAL ET AL: "Development of a Potent Wound Healing Agent Based on the Liver Fluke Granulin Structural Fold", JOURNAL OF MEDICINAL CHEMISTRY, vol. 60, no. 10, 3 May 2017 (2017-05-03), pages 4258-4266, XP055469901, US ISSN: 0022-2623, DOI: 10.1021/acs.jmedchem.7b00047
- SMOUT, MICHAEL J. ET AL.: 'A granulin-like growth factor secreted by the carcinogenic liver fluke, Opisthorchis viverrini, promotes proliferation of host cells' PLOS PATHOGENS vol. 5, no. 10, 2009, pages 1 - 16, XP055192406
- SMOUT, MICHAEL J. ET AL.: 'Carcinogenic parasite secretes growth factor that accelerates wound healing and potentially promotes neoplasia' PLOS PATHOGENS vol. 11, no. 10, 2015, pages 1 - 20, XP055469897
- BANSAL, PARAMJIT S. ET AL.: 'Development of a potent wound healing agent based on the liver fluke granulin structural fold' JOURNAL OF MEDICINAL CHEMISTRY vol. 60, no. 10, 2017, pages 4258 - 4266, XP055469901

## Description

### TECHNICAL FIELD

This invention relates to an isolated peptide and an isolated nucleic acid and their use as a medicament, particularly for the treatment of wounds, a use of such an isolated peptide or nucleic acid for in vitro promoting in vitro cell proliferation and migration, a method of promoting in vitro cell proliferation and/or migration, a genetic construct, a host cell, and a pharmaceutical composition.

### BACKGROUND

Granulins are a family of protein growth factors involved in a wide range of physiological functions and disease processes including embryogenesis, wound repair, inflammation and tumour growth¹. The human parasitic liver fluke *Opisthorchis viverrini* secretes a granulin family member called *Ov*-GRN-1, which was originally isolated from the excretory/secretory (ES) products of the carcinogenic trematode^{2, 3}. *Ov*-GRN-1 was the first growth factor described from a pathogen to cause proliferation of host cells^{4, 5}. We have shown that picomolar concentrations of recombinant Ov-GRN-1 induce angiogenesis and accelerate wound repair in mice upon topical administration, findings that indicate that liver fluke granulin might be developed as a treatment for wounds⁶.

An understanding of the structure-activity relationship for *Ov*-GRN-1 would enable design of the most efficacious form of this granulin for healing wounds. The three-dimensional structure of *Ov*-GRN-1 has not been experimentally determined, but structures for granulins of several species have been reported. The initial granulin structure determined was that of carp granulin-1; this comprises four β-hairpins crosslinked together by six disulfide bonds in a ladder-shaped arrangement of the disulfide bonds⁷. Despite the well-defined structure observed for carp granulin-1, the structure function relationships of granulins are complex and appear to be highly dependent on the primary sequence. This is particularly evident with the human granulins. The precursor protein of mammalian granulin (progranulin, PGRN) contains seven-and-a-half granulin domains that are approximately 6 kDa in molecular mass and are proteolytically processed into individual granulin modules after secretion of PGRN from the cell¹. The "half-granulin" unit, termed paragranulin, contains only six cysteine residues⁸.

The seven human granulin modules have been expressed individually and the structures analyzed by NMR spectroscopy⁹. Three contain relatively well-defined three-dimensional structures in solution (A, C and F), whereas the others are mainly mixtures of poorly structured disulfide isomers⁹. The structure of human granulin A includes a β-hairpin structure similar to carp granulin-1 but there is significant structural disorder in the C-terminal region. Of the well folded human granulin modules, granulin A demonstrates potent inhibition of proliferation of a breast cancer cell line, while by contrast, human granulin F stimulates cell proliferation⁹. The poorly folded peptides exhibit weak or no inhibitory or activity. It should be noted, however, that the limited activity may be due to the absence of key signaling pathways in the target cells, and/or that the production of the recombinant peptides in bacteria induced incomplete/incorrect folding. To date, the range of granulin activities and binding partners is broad, and seemingly organ- and co-factor-dependent¹⁰⁻¹³.

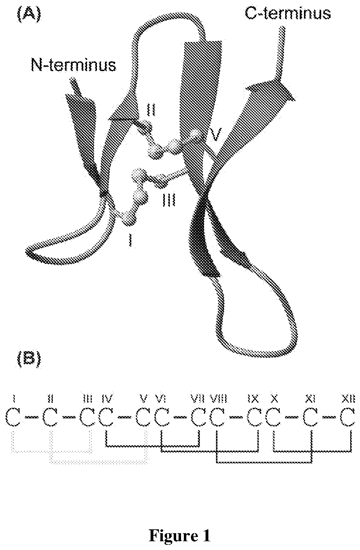

Structural analysis with NMR spectroscopy has shown that the N-terminal regions of carp granulin-1 and human granulin A can fold independently of the C-terminal regions^{14, 15}. Truncated analogues of these two granulins containing only two disulfide bonds, have β-hairpin structures, as shown for a 30-residue N-terminal domain of carp granulin-1 (Figure 1).

M. J. Smout et al. (PLoS Pathog. 5(10), 1-16, 2009) describe a granulin-like growth factor secreted by the Southeast Asian liver fluke (Opisthorchis viverrini), promoting proliferation of host cells. Chronic infection with the parasitic worm Opisthorchis viverrini is a major cause of cholangiocarcinoma. One mechanism is the secretion of parasite proteins with mitogenic properties into the bile ducts, driving cell proliferation and creating a tumorigenic environment. A homologue of human granulin has been found in the excretory/secretory products of the parasite, namely O. viverrini granulin, termed Ov-GRN-1.

M. J. Smout et al. (PLoS Pathog. 11(10), 1-20, 2015) describe that O. viverrini granulin growth factor, Ov-GRN-1, accelerates wound healing and blood vessel growth, but also leads to a carcinogenic microenvironment.

A Granulin-like protein found in Clonorchis sinensis (Chinese liver fluke) is disclosed in the UniProt database (XP002797809; EBI accession no. UNIPFIOT:G7Y9L4) WO 93/15195 A1 discloses cystine rich leukocyte peptides with a size of approx. 5 kDa that have wound healing effects.Kda.

W.F. Vranken et al. (J. Pept. Res. 53(5), 590-597, 1999) describe a 30-residue fragment of carp granulin-1 that folds into a stack of two beta-hairpins, the same conformation as formed within the native protein. M.J. Smout et al. (Protein Expr. Purif. 79(2), 263-70, 2011) describe expression, refolding and purification of ov-GRN-1 growth factor.

P. S. Bansal et al. (J. Med Chem 60(10), 4258-4266, 2017) describe N-terminal analogues of Ov-GRN-1 and their use as wound healing agents.

### SUMMARY

Surprisingly, peptides derived from the N-terminal region of granulin have activity in promoting cell proliferation, migration and/or wound healing. Furthermore, an additional disulphide bond may be engineered into such peptides to improve or otherwise modify the folding of the peptide.

The invention provides an isolated peptide comprising an amino acid sequence derived from, or based on, the amino acid sequence of an N-terminal region of a granulin protein, as defined in the claims.

In one aspect, the invention provides an isolated peptide consisting of the amino acid sequence:
¹X*ₙ* C ²XD ³XVYTCR ⁴XGQTC C/A RGLHGYGC ⁵X*ₘ* (SEQ ID NO:1), wherein ¹Xₙis a sequence of amino acids of length n, with n = 0-11 and ¹X being any amino acid; wherein each of ²X, ³X, and ⁴X is P or A; wherein ⁵Xₘ is a sequence of amino acids of length m, with m = 0-4 and ⁵X being any amino acid; and wherein the isolated peptide comprises, or is capable of forming, two or three intrachain disulphide bonds.

In one embodiment, when n is 3, ¹Xₙ = SPS

In one embodiment, when n is 4, ¹Xₙ = RSPS

In one embodiment, when n is 11, ¹Xₙ = MDTLQPIRSPS

In one embodiment, when m is 1, ⁵Xₘ = C or A

In one embodiment, when m is 4, ⁵Xₘ = APMD

In another embodiment, when m is 4, ⁵Xₘ = CPMD

In some preferred embodiments, at least one, at least two, or each of ²X ³X ⁴X = P.

In some preferred embodiments, one of ²X ³X ⁴X = A.

In a related aspect, the invention provides an isolated peptide that consists of the amino acid sequence set forth in any one of FIGS 1-13 or Table 3 other than SEQ ID NO:11.

In a particular embodiment, the isolated peptide consists of an amino acid sequence set forth in any one of SEQ ID NOS:1-10.

The isolated peptide according to the invention comprises, or is capable of forming, two or three intrachain disulphide bonds.

In some embodiments of the aforementioned aspects, the isolated peptide is capable of forming proline *cis*/*trans* isomers.

In a second aspect of the disclosure that is not part of the invention, there is provided a method of modifying a peptide comprising an amino acid sequence set forth in any one of SEQ ID NOS:1-11, or an amino acid sequence at least 70% identical thereto, including the step of incorporating one or more amino acid insertions, deletions, or substitutions into the amino acid sequence of the peptide.

Preferably, modifying the peptide results one or more increased or enhanced biological activities of the peptide.

A third aspect of the disclosure provides an isolated peptide modified according to the method of the second aspect.

Also provided, but not part of the claimed invention, is an antibody or antibody fragment that specifically binds the isolated peptide of the first or third aspects.

In another aspect, the invention provides an isolated nucleic acid encoding the isolated peptide according to the invention.

In yet another aspect, the invention provides a genetic construct comprising the isolated nucleic acid of the aforementioned nucleic acid.

In still yet another aspect, the invention provides a host cell comprising the genetic construct of the aforementioned aspect.

In a further aspect, the invention provides a pharmaceutical composition comprising the isolated peptide or the isolated nucleic acid of the aforementioned aspects together with a pharmaceutically acceptable carrier, diluent or excipient. Furthermore, the invention provides an isolated polypeptide according to the invention for use as a medicament, as well as an isolated nucleic acid according to the invention for use as a medicament.

In addition, the invention provides an isolated polypeptide according to the invention for use in the treatment of wounds, as well as an isolated nucleic acid according to the invention for use in the treatment of wounds.

Yet another aspect of the invention comprises the use of an isolated polypeptide according to the invention, or an isolated nucleic acid according to the invention, or a or a genetic construct according to the invention, or a pharmaceutical composition according to the invention, for initiating, promoting, stimulating, or facilitating in vitro cell proliferation and/or in vitro cell migration.

Yet a further aspect of the invention comprises the use of an isolated polypeptide according to the invention, or an isolated nucleic acid according to the invention, or a pharmaceutical composition according to the invention, for initiating, promoting, stimulating, or facilitating in vitro cell proliferation and/or in vitro cell migration.

In another further aspect, the invention provides a method of promoting in vitro cell proliferation and/or migration, said method including the step of contacting one or more cells with the isolated peptide, isolated nucleic acid or the pharmaceutical composition of the aforementioned aspects to thereby initiate, stimulate or facilitate proliferation and/or migration of the one or more cells.

In yet another further aspect of the disclosure that is not part of the invention, a method is provided of healing a wound, said method including the step of contacting the wound with the isolated peptide, isolated nucleic acid or the pharmaceutical composition of the aforementioned aspects to thereby at least partly heal the wound.

In still yet another further aspect of the disclosure that is not part of the invention, a method is provided of producing an agent that promotes cell proliferation, migration and/or heals wounds, said method including the step of identifying, engineering, screening or designing an analogue or agonist of the isolated peptide of the aforementioned aspects that promotes cell proliferation and/or migration and/or heals wounds.

This aspect of the disclosure also provides an agent that promotes cell proliferation, migration and/or heals wounds produced by the method of this aspect.

Suitably, the agent may be used according to the methods of the aforementioned aspects.

Throughout this specification, unless otherwise indicated, *"comprise", "comprises"* and *"comprising"* are used inclusively rather than exclusively, so that a stated integer or group of integers may include one or more other non-stated integers or groups of integers.

By *"consist essentially of"* is meant in this context that the isolated protein or each immunogenic fragment has one, two or no more than three amino acid residues in addition to the recited amino acid sequence. The additional amino acid residues may occur at the N- and/or C-termini of the recited amino acid sequence, although without limitation thereto.

It will also be appreciated that the indefinite articles "a" and *"an"* are not to be read as singular indefinite articles or as otherwise excluding more than one or more than a single subject to which the indefinite article refers. For example, "a" protein includes one protein, one or more proteins or a plurality of proteins.

### BRIEF DESCRIPTION OF THE FIGURES

**Figure 1****. Three-dimensional structure of a 30 residue N-terminal domain of carp granulin-1.** (A) PDB code 1QGM, the β-strands are shown as purple arrows and the disulfide bonds in yellow ball and stick format. The image was generated using MolMol. (B) The disulfide connectivity in the full length carp granulin-1 protein⁷. Cysteines are designated with sequential roman numerals I-XII. The two bonds in the truncated carp granulin-1 are highlighted in yellow.

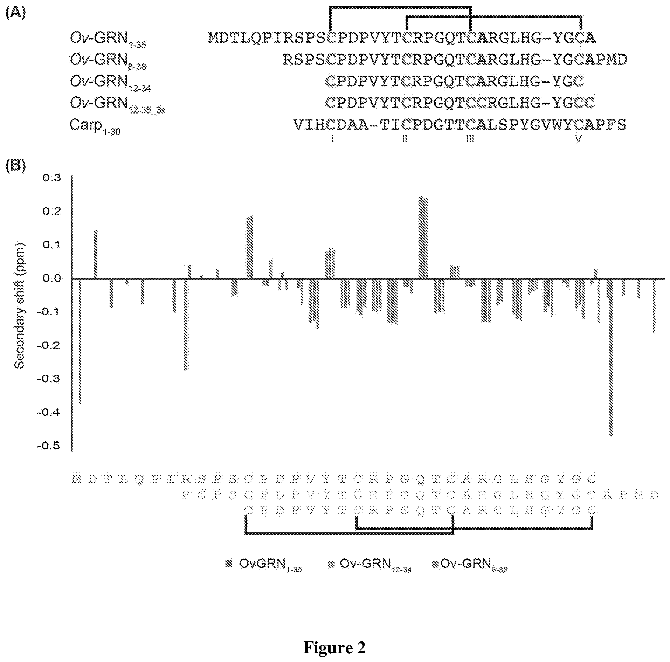

**Figure 2****. Sequences and secondary shifts of the *Ov*-GRN-1 truncated analogues.** (A) Sequences show CysIV and CysVI were replaced with alanine residues; the cysteines are highlighted in red and the substitutions are shown in blue. The N-terminal 30 residues of carp granulin-1 is also provided, with CysIV and VI replaced with alanine residues. Sequence identifiers are as follows: *Ov*-GRN(1-35) = SEQ ID NO:2; *Ov*-GRN(8-38) = SEQ ID NO:3; *Ov*-GRN(12-34) = SEQ ID NO:4; AND *Ov-*GRN(12-35) = SEQ ID NO:5. (B) Secondary shifts of *Ov*-GRN-1 peptides with four cysteine residues (*Ov*-GRN₁₋₃₅, *Ov*-GRN₈₋₃₈ and *Ov*-GRN₁₂₋₃₄). The secondary shifts were derived by subtracting random coil shifts¹⁶ from the αH shifts. The similarity in the secondary shifts for the conserved residues indicates that the overall fold is the same in the three peptides. Color scheme is retained in following figures. Both panels: black connecting lines represent disulfide bond connectivity.

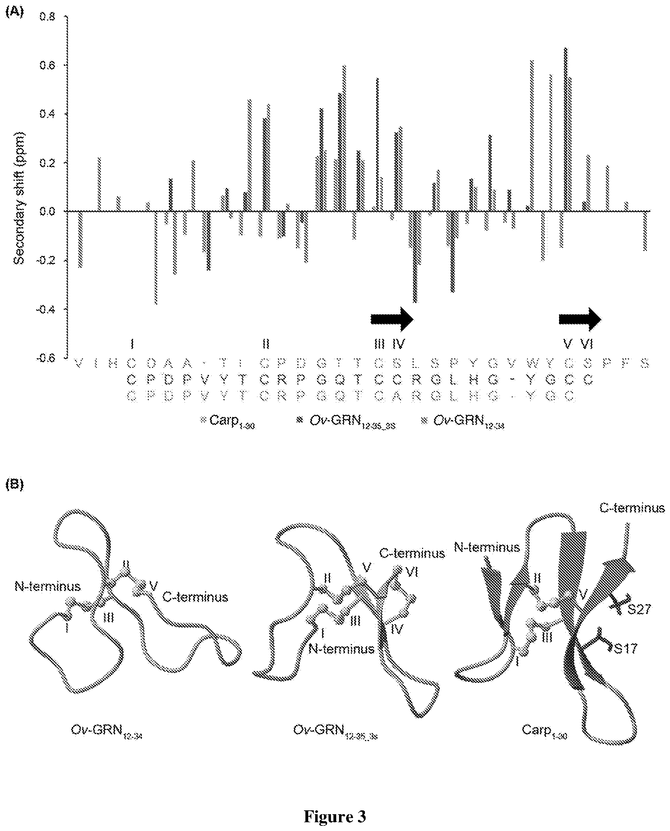

**Figure 3****: Structural analysis of *Ov*-GRN₁₂₋₃₄ and *Ov*-GRN_{12-35_3s}.** (A) Secondary shifts of *Ov*-GRN₁₂₋₃₄ and *Ov*-GRN_{12-35_3s} compared to carp granulin₁₋₃₀. The secondary shifts were derived by subtracting random coil shifts¹⁶ from the αH shifts. *Ov*-GRN₁₂₋₃₄ has significantly different secondary shifts compared to *Ov*-GRN_{12-35_3s} and carp₁₋₃₀, and lacks positive shifts indicating a lack of β-sheet structure. Despite the differences in sequence the trends for the secondary shifts between *Ov*-GRN_{12-35_3s} and carp₁₋₃₀ are similar indicating that the β-sheet present in carp granulin-1 is also present in *Ov*-GRN_{12-35_3s} (black arrows). (B) The structures of *Ov*-GRN₁₂₋₃₄ and Ov-GRN_{12-35_3s} were determined using NMR spectroscopy and confirms that *Ov*-GRN₁₂₋₃₄ does not contain β-sheet structure but *Ov*-GRN_{12-35_3s} does (blue arrows). Disulfide bonds are shown as yellow ball and stick representations and the structure of carp₁₋₃₀ are shown for comparison. The side-chains of residues Ser17 and Ser27 are highlighted on the carp₁₋₃₀ structure, to indicate the Cys-Ser substituted sites of CysIV and Cys VI. Based on this structure it appears likely that CysIV and CysVI of carp₁₋₃₀ could form a disulfide bond, consistent with the likely connectivity in *Ov*-GRN_{12-35_3s}.

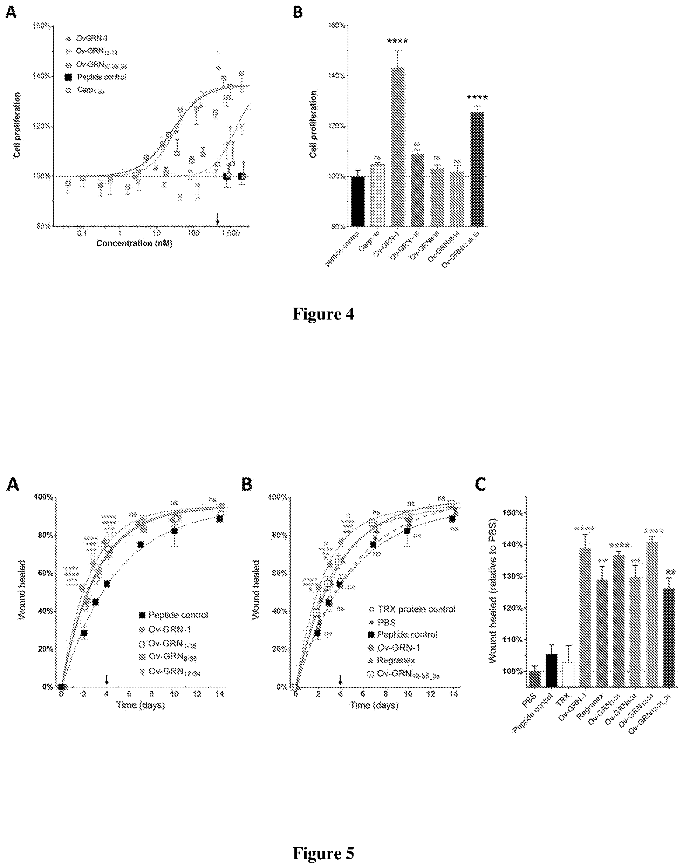

**Figure 4****. Liver fluke granulin peptides induce cell proliferation.** (A) *Opisthorchis viverrini* granulin peptides but not carp granulin₁₋₃₀ induced proliferation of H69 human cholangiocytes at a range of concentrations as monitored using xCELLigence. Only selected treatments are graphed to aid visualization. Variable slope dose response lines of best fit show proliferation four days after a single application of treatment. *Ov*-GRN_{12-35_3s} potency characterized by significantly increased cell proliferation observed at final concentrations of ≥15 nM (p<0.05). Black arrow denotes 400-483 nM concentration used in panel B. (B) Mean proliferation at 400 nM of all Ov-GRN-1 synthesized peptides and 483 nM *Ov*-GRN-1 protein from panel A. ns = not significant, ^{∗∗∗∗}p<0.0001. Both Panels: 2-way ANOVA test with Dunnett's correction for multiple comparisons was used to compare treatments with relevant treatment controls (*Ov*-GRN-1 protein relative to thioredoxin expression matched recombinant protein control and peptides relative to peptide control). Mean values from 4-6 replicates pooled from 2-4 experiments with SEM bars shown either above or below for clarity.
**Figure 5****. Mouse wound healing activity of *Ov*-GRN-1 and peptides.** (A+B) Wound healing outcomes from treatments with 56 pmoles of recombinant *Ov*-GRN-1, Ov-GRN-1 peptides, unrelated peptide, thioredoxin (TRX) protein controls and 71 pmoles Regranex in 1.5% methylcellulose gel applied daily in 50 µl volume from days 0-4 to a ~0.2 cm² wound arising from biopsy punch to the scalp between the ears. To aid visualisation, data were split across two graphs with the *Ov*-GRN-1 and peptide control groups shown in both panels. No significant differences between the unrelated peptide control, PBS, or TRX protein control were noted at any time point. Black arrows denote the day 4 time point used in panel C. (C) Wound healing relative to PBS vehicle control from day 4. All panels: mean healing rates of 2-6 biological replicates of groups of 4-5 animals plotted with SEM bars. Groups have been marginally shifted left or right to aid viewing. Repeated measure 2-way ANOVA test with Dunnett's correction for multiple comparisons compare each group against each other group. Significance against peptide/protein control signified by ^{∗∗∗∗} = p<0.0001, ^{∗∗∗} = p<0.001, ^{∗∗} = p<0.01, ^{∗} = p<0.05, ns=not significant. Significant treatments against Regranex signified by # = p<0.05. Color of asterisk or hash represents the relevant group. The colors and symbols are maintained across Figures 2-5.

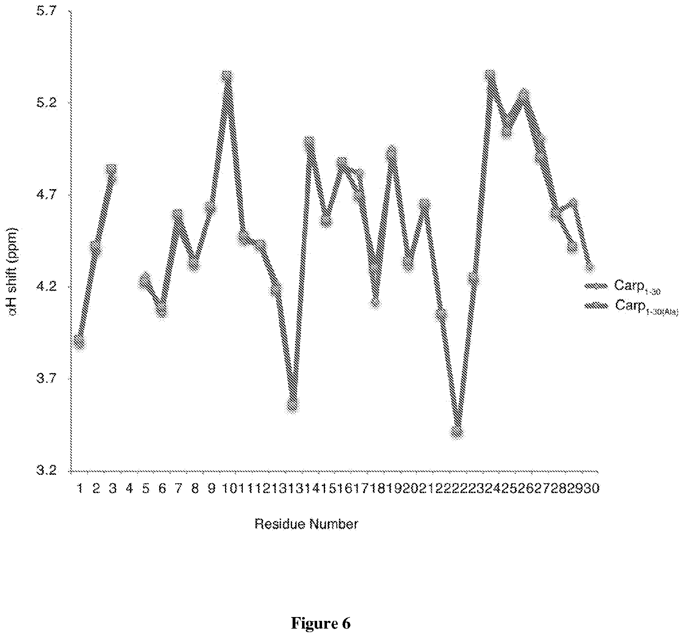

**Figure 6****. Chemical shift comparison.** Chemical shift comparison between the published shifts of a truncated form of carp granulin¹⁴ and the mutant with C17A and C27A mutations.

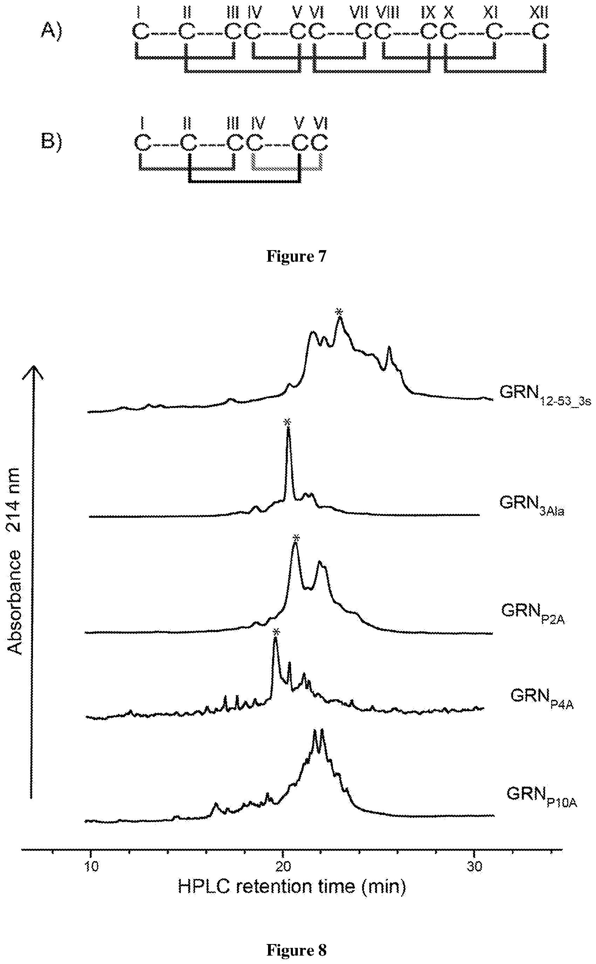

**Figure 7****. Conserved cysteine framework in granulin family.** A) Disulfide bond connectivity pattern in Carp-1 granulin. (B) Disulfide bond connectivity pattern in the synthetic N-terminal *Ov*-GRN-1 peptide (GRN _{12-35_3s}). Non-native disulfide bond in GRN _{12-35_3s} based on the predicted connectivity between Cys IV and Cys VI is highlighted with red colour; "---" could be any residues.
**Figure 8****. HPLC trace of engineered peptides.** All peptides were oxidised upon free air oxidation for 24 hour at room temperature. Collected fractions correspond to the oxidised peptide with the expected molecular mass according to the MALDI spectroscopy analyses are marked with ^{∗}.

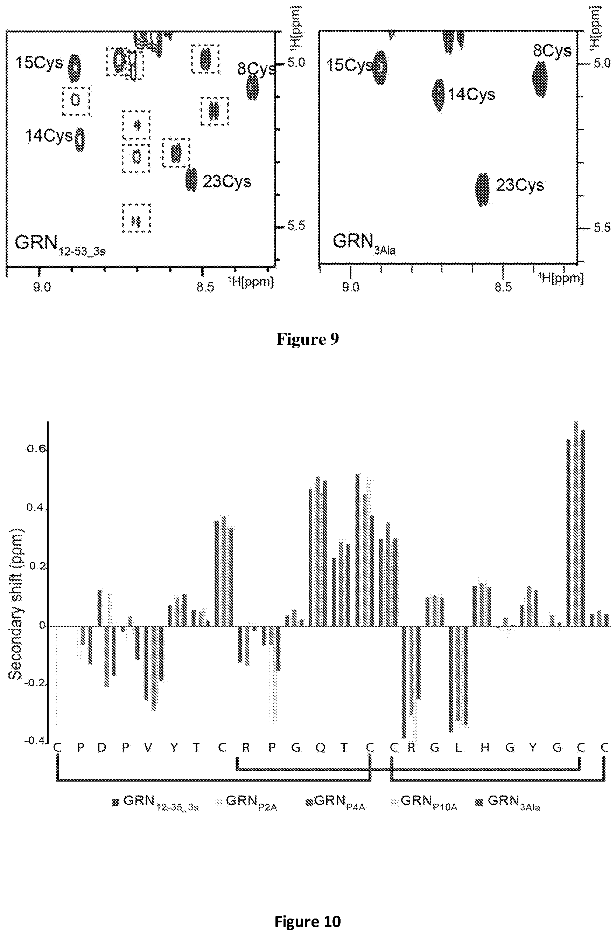

**Figure 9****. TOCSY Two-dimensional NMR spectrum of GRN_{12-35_3s} compared to GRN_{3Ala} in NH region.** The TOCSY spectrum was recorded at 0.2 mM concentration, 290K and mixing time of 80ms. Assigned residues are shown with their residue name and number. Additional peaks that arise from adopting multiple confirmations by GRN_{12-35_3s} are boxed.
**Figure 10****. Secondary shifts of *Ov*-GRN_{12-35_3s} engineered peptides compared to *Ov*-GRN_{12-35_3s}**.The secondary shifts were derived by subtracting random coil shifts from the αH shifts. The trends for the secondary shifts are similar compared to Ov-GRN_{12-35_3s} indicating that the β-sheet present in *Ov*-GRN_{12-35_3s} is maintained in proline mutant analogues.

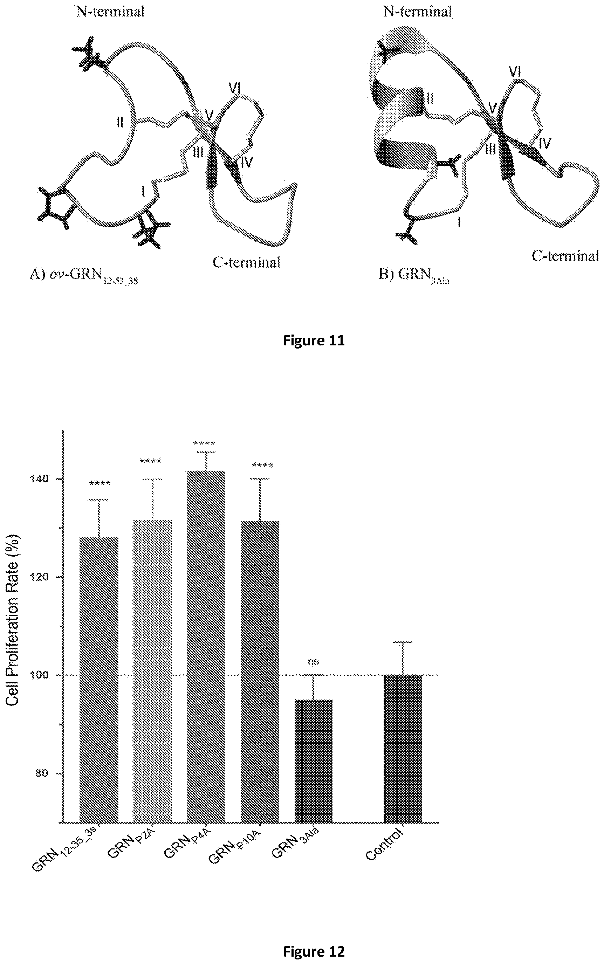

**Figure 11****. Three dimensional structure of *Ov-*GRN_{12-35_3s} (left) and GRN_{3Ala} (right).** The structures were determined using NMR spectroscopy .The side-chains of Proline 2, Proline 4 and Proline 10 residues are highlighted on the *Ov*-GRN_{12-35_3s}. Proline resides are replaced with alanine, which the side chains are highlighted, in GRN_{3Ala} .
**Figure 12****. Effect of engineered *Ov*-GRN_{12-35_3s} peptides on in vitro cell proliferation of H69 cells.** The H69 cells were treated with 200 nM peptide concentrations for 48 h. The proliferation rates of peptides were measured as described in materials and methods, and the values are given as percentage. Data were analyzed by one-way ANOVA. Significance was set at *ns P<0.05,* ^{∗∗} *P*<0.001, ^{∗∗∗} *P*<0.001 ^{∗∗∗∗}*P*<0.0001. Statistically different values of *P*<0.0001(*Ov*-GRN_{12-35_3s}, GRN_{P2A}, GRN_{P4A} and GRN_{P10A}) and *P* = 0.90 (GRN3Ala) were determined compared with the control peptide Loop6.

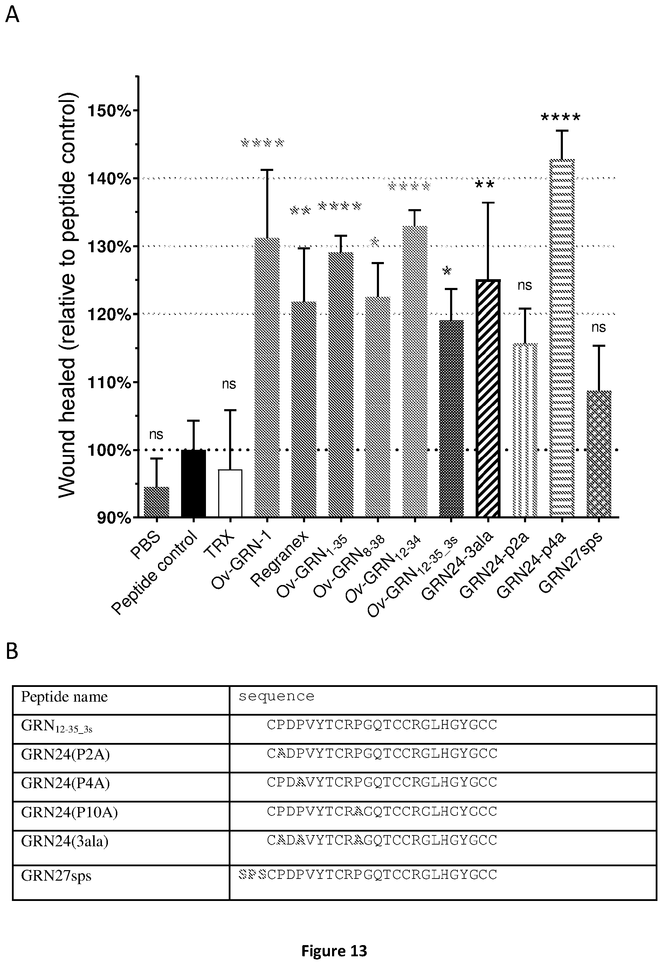

**Figure 13****. Mouse wound healing activity of *Ov*-GRN-1 and peptides.** (A) Day 4 wound healing outcomes are shown relative to 56 pmoles peptide control from treatments with 56 pmoles of recombinant *Ov*-GRN-1, *Ov*-GRN-1 peptides, thioredoxin (TRX) protein controls and 71 pmoles Regranex in 1.5% methylcellulose gel applied daily in 50 µl volume from days 0-4 to a ~0.2 cm² wound arising from biopsy punch to the scalp between the ears. (B) Amino acid sequences of peptides. GRN27sps = SEQ ID NO: 10.

### BRIEF DESCRIPTION ON THE SEQUENCE LISTING

| | |
|---|---|
| SEQ ID NO:1 | Amino acid sequence of truncated *Ov*-GRN-1 peptide |
| SEQ ID NO:2 | Amino acid sequence of *Ov*-GRN(1-35) peptide. |
| SEQ ID NO:3 | Amino acid sequence of *Ov*-GRN(8-38) peptide. |
| SEQ ID NO:4 | Amino acid sequence of *Ov*-GRN(12-34) peptide. |
| SEQ ID NO:5 | Amino acid sequence of *Ov*-GRN(12-35) peptide. |
| SEQ ID NO:6 | Amino acid sequence of GRN(P2A) peptide. |
| SEQ ID NO:7 | Amino acid sequence of GRN(P4A) peptide. |
| SEQ ID NO:8 | Amino acid sequence of GRN(P10A) peptide. |
| SEQ ID NO:9 | Amino acid sequence of GRN(3Ala) peptide. |
| SEQ ID NO: 10 | Amino acid sequence of GRN27sps. |
| SEQ ID NO: 11 | Amino acid sequence of Carp(1-30) peptide. |

### DETAILED DESCRIPTION

The present invention is at least partly predicated on the synthesis of truncated versions of *Ov*-GRN-1, and/or variants thereof, determining the folding properties of these and their activity in cell proliferation and wound healing. The N-terminal region of *Ov*-GRN-1 displayed novel folding properties, and potent cell proliferation activity. Some such truncated peptides and variants have been tested in a mouse model of wound healing and are as potent as the full-length protein and as potent, or even superior to, Regranex, a clinically used wound-healing agent.

Accordingly, an aspect of the invention relates to an isolated peptide consisting of the amino acid sequence of SEQ ID NO: 1.

For the purposes of this invention, by *"isolated"* is meant material that has been removed from its natural state or otherwise been subjected to human manipulation. Isolated material may be substantially or essentially free from components that normally accompany it in its natural state, or may be manipulated so as to be in an artificial state together with components that normally accompany it in its natural state. Isolated material may be in native, chemical synthetic or recombinant form.

By *"protein"* is meant an amino acid polymer. The amino acids may be natural or non-natural amino acids, D- or L-amino acids as are well understood in the art.

The term *"protein"* includes and encompasses *"peptide",* which is typically used to describe a protein having no more than fifty (50) amino acids and *"polypeptide",* which is typically used to describe a protein having more than fifty (50) amino acids. An embodiment of the invention provides an isolated polypeptide comprising the amino acid sequence of SEQ ID NO:1 or an amino acid sequence at least 70% identical thereto.

The isolated peptide according to the invention consists of the amino acid sequence:
¹X*ₙ* C ²XD ³XVYTCR ⁴XGQTC C/A RGLHGYGC ⁵X*ₘ* (SEQ ID NO:1) wherein n is 0-11 and *m* is 0-4.
¹ Xₙ is a sequence of amino acids of length n, with n = 0-11 and ¹X being any amino acid. It will be appreciated that when n is zero, there is no amino acid present.

Each of ²X, ³X, and ⁴X is P or A.

In one embodiment, when *n* is 4, ¹Xₙ = RSPS

In one embodiment, when *n* is 3, ¹Xₙ = SPS

In one embodiment, when *n* is 11, ¹Xₙ = MDTLQPIRSPS
⁵Xₘ is a sequence of amino acids of length m, with m = 0-4 and ⁵X being any amino acid. It will be appreciated that when m is zero, there is no amino acid present.

In one embodiment, when *m* is 1, ⁵Xₘ = C or A.

In one embodiment, when *m* is 4, ⁵Xₘ = APMD.

In another embodiment, when *m* is 4, ⁵Xₘ = CPMD

Each of ²X ³X ⁴X is independently P or A.

In some preferred embodiments, at least one, at least two, or each of ²X ³X ⁴X = P.

In some preferred embodiments, one of ²X ³X ⁴X = A.

The isolated peptide comprises, or is capable of forming, two or three intrachain disulphide bonds.

In particular embodiments, the isolated peptide consists of the amino acid sequence set forth in any one of SEQ ID NOS:1-10.

Isolated peptides consisting of the respective amino acid sequences of SEQ ID NOS:2-5 may be referred to herein as:

| | |
|---|---|
| SEQ ID NO:2 | *Ov*-GRN₁₋₃₅, *Ov*-GRN(1-35), or GRN₁₋₃₅; |
| SEQ ID NO:3 | *Ov*-GRN₈₋₃₈, *Ov*-GRN(8-38), or GRN₈₋₃₈; |
| SEQ ID NO:4 | *Ov*-GRN₁₂₋₃₄, *Ov*-GRN(2-24), or GRN₂₋₂₄; |
| SEQ ID NO:5 | *Ov*-GRN_{12-35_3s}, *Ov*-GRN(12-35), or GRN_{12-35_3s} |

With reference to the Examples and FIGS. 4 and 5, isolated peptides consisting of SEQ ID NOS:2-5 were demonstrated to be capable of promoting cell proliferation and/or wound healing to a degree or level as higher or higher than Regranex.

In some embodiments, the isolated peptide comprises two or three intrachain disulphide bonds. As will be appreciated from the amino acid sequences shown in FIG.2, SEQ ID NOS:2-4 comprise four (4) cysteine residues that form two (2) disulphide bonds, while SEQ ID NO:5 comprises six (6) cysteine residues that form three (3) disulphide bonds. Although not wishing to be committed to any particular theory, it is proposed that the isolated peptide of SEQ ID NO:5 may fold in a manner similar to a full length granulin protein.

Isolated peptides consisting of the respective amino acid sequences of SEQ ID NOS:6-8 may be referred to herein as:

| | |
|---|---|
| SEQ ID NO:6 | *Ov*-GRN_{P2A}, *Ov*-GRN(P2A), GRN_{P2A}, GRN24(P2A); |
| SEQ ID NO:7 | *Ov*-GRN_{P4A}, *Ov*-GRN(P4A), GRN_{P4A}, GRN24(P4A); |
| SEQ ID NO:8 | *Ov*-GRN_{P10a}, *Ov*-GRN(P10A), GRN_{P10A}, GRN(P 10A); |

Each of the amino acid sequences of SEQ ID NOS:6-8 is a form of SEQ ID NO:1 wherein one of ²X ³X ⁴X is alanine, and the remaining of ²X ³X ⁴X are proline. With reference to the Examples and Table 3, each of the amino acid sequence of SEQ ID NOS:6-8 is also variant of the amino acid sequence of SEQ ID NO:5, wherein a respective single proline residue of SEQ ID NO:5 has been substituted with an alanine residue.

In another embodiment, the isolated peptide comprises, consists essentially of, or consists of the amino acid sequence set forth in SEQ ID NO:9, or fragments or variants thereof. An isolated peptide consisting of the amino acid sequence of SEQ ID NO:9 may be referred to herein as *Ov*-GRN_{3Ala}, *Ov*-GRN(3Ala), or GRN_{3Ala}. The amino acid sequence of SEQ ID NO:9 is a form of SEQ ID NO: 1 wherein each of ²X ³X ⁴X is alanine. With reference to the Examples and Table 3, SEQ ID NO:9 is also variant of the amino acid sequence of SEQ ID NO:5, wherein each of the three proline residues of SEQ ID NO:5 has been substituted with an alanine residue.

As set forth in the Examples and FIG. 12, isolated peptides with the amino acid sequences of SEQ ID NOS:6-8 enhanced cell proliferation at the same or greater rates than a peptide with the amino acid sequence of SEQ ID NO:5. However, an isolated peptide with the amino acid sequence of SEQ ID NO:9 did not enhance cell proliferation.

Accordingly, in some preferred embodiments, the isolated peptide comprises the amino acid sequence set forth in SEQ ID NO: 1 wherein at least one, but preferably less than three, of²X ³X ⁴X is alanine. Preferably one of²X ³X ⁴X is alanine. Preferably at least one of ²X ³X ⁴X is proline.

It will be further appreciated, with reference to the Examples and Figures 9-10, that peptides with the amino acid sequences of SEQ ID NOS:5-8 demonstrated multiple conformations that were absent in a peptide with the amino sequence SEQ ID NO:9. Without being bound by theory, it is believed that these multiple conformations are the result of formation of proline *cis*/*trans* isomers in SEQ ID NOS:5-8, but not in SEQ ID NO:9. These multiple conformations may contribute to the observed biological activity present in peptides with the amino acid sequences of SEQ ID NOS:5-8, but absent in a peptide with the amino acid sequence of SEQ ID NO:9.

Accordingly, in some preferred embodiments, the isolated peptide is capable of forming proline *cis*/*trans* isomers.

SEQ ID NO:10 comprises an N-terminal amino acid sequence SPS. SEQ ID NO:10 may be referred to herein as *Ov*-GRNₛₚₛ, *Ov*-GRN(SPS), GRNₛₚₛ, or GRN27sps.

The disclosure also provides isolated peptides comprising an amino acid sequence at least 70% identical to any one of SEQ ID NOS:1-10, referred to herein as a peptide "variant".

As used herein, a peptide *"variant"* has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity with the amino acid sequence set forth in any one of SEQ ID NOS: 1-10. The peptide *"variant"* disclosed herein may have one or more amino acids deleted or substituted by different amino acids. It is well understood in the art that some amino acids may be substituted or deleted without changing biological activity of the peptide (conservative substitutions). Suitably, the variant has at least 50%, 55%, 60%, 65%, 70%, 75%, 80%, 85%, 90%, 91%, 92%, 93%, 94%, 95%, 96%, 97%, 98% or 99% of the biological activity of the isolated peptide of any one of SEQ ID NOS:1-10. In particular embodiments, the variant comprises, or is capable of forming, two or three intrachain disulphide bonds.

Terms used generally herein to describe sequence relationships between respective proteins and nucleic acids include *"comparison window", "sequence identity", "percentage of sequence identity"* and *"substantial identity".* Because respective nucleic acids/proteins may each comprise (1) only one or more portions of a complete nucleic acid/protein sequence that are shared by the nucleic acids/proteins, and (2) one or more portions which are divergent between the nucleic acids/proteins, sequence comparisons are typically performed by comparing sequences over a "comparison window" to identify and compare local regions of sequence similarity. A *"comparison window"* refers to a conceptual segment of typically 6, 9 or 12 contiguous residues that is compared to a reference sequence. The comparison window may comprise additions or deletions (*i.e.,* gaps) of about 20% or less as compared to the reference sequence for optimal alignment of the respective sequences. Optimal alignment of sequences for aligning a comparison window may be conducted by computerised implementations of algorithms (Geneworks program by Intelligenetics; GAP, BESTFIT, FASTA, and TFASTA in the Wisconsin Genetics Software Package Release 7.0, Genetics Computer Group, 575 Science Drive Madison, WI, USA) or by inspection and the best alignment (*i.e.* resulting in the highest percentage homology over the comparison window) generated by any of the various methods selected. Reference also may be made to the BLAST family of programs as for example disclosed by Altschul et al., 1997, Nucl. Acids Res. 25 3389. A detailed discussion of sequence analysis can be found in Unit 19.3 of CURRENT PROTOCOLS IN MOLECULAR BIOLOGY Eds. Ausubel et al. (John Wiley & Sons Inc NY, 1995-2015).

The term *"sequence identity"* is used herein in its broadest sense to include the number of exact nucleotide or amino acid matches having regard to an appropriate alignment using a standard algorithm, having regard to the extent that sequences are identical over a window of comparison. Thus, a *"percentage of sequence identity"* is calculated by comparing two optimally aligned sequences over the window of comparison, determining the number of positions at which the identical nucleic acid base (*e.g.,* A, T, C, G, I) occurs in both sequences to yield the number of matched positions, dividing the number of matched positions by the total number of positions in the window of comparison (*i.e.,* the window size), and multiplying the result by 100 to yield the percentage of sequence identity. For example, *"sequence identity"* may be understood to mean the "match percentage" calculated by the DNASIS computer program (Version 2.5 for windows; available from Hitachi Software engineering Co., Ltd., South San Francisco, California, USA).

The disclosure also provides fragments of the isolated peptide disclosed herein. In some embodiments, fragments may comprise, consist essentially of, or consist of 6, 7, 8, 9, 10, 11, 12, 13, 14, 15, 16, 17, 18, 19, 20, 21, 22, 23, 24, 25, 26, 27, 28, 29, 30, 31, 32, 33 or 34 amino acids of any one of SEQ ID NOS:1-10. In particular embodiments, the fragments comprise, or are capable of forming, two or three intrachain disulphide bonds.

Suitably, the fragments are biologically active. Preferably, the fragment has at least 50%, 55%, 60%, 65%, 70%, 75%, 80%, 85%, 90%, 91%, 92%, 93%, 94%, 95%, 96%, 97%, 98% or 99% of the biological activity of the isolated peptide of any one of SEQ ID NOS:1-10.

Derivatives of the isolated peptide disclosed herein are also provided. As used herein, *"derivative"* proteins or peptides have been altered, for example by conjugation or complexing with other chemical moieties, by post-translational modification (e.g. phosphorylation, ubiquitination, glycosylation), chemical modification (e.g. cross-linking, acetylation, biotinylation, oxidation or reduction and the like), conjugation with labels (e.g. fluorophores, enzymes, radioactive isotopes) and/or inclusion of additional amino acid sequences as would be understood in the art.

In this regard, the skilled person is referred to Chapter 15 of CURRENT PROTOCOLS IN PROTEIN SCIENCE, Eds. Coligan et al. (John Wiley & Sons NY 1995-2015) for more extensive methodology relating to chemical modification of proteins.

Additional amino acid sequences may include fusion partner amino acid sequences which create a fusion protein. By way of example, fusion partner amino acid sequences may assist in detection and/or purification of the isolated fusion protein. Non-limiting examples include metal-binding (*e*.*g*. polyhistidine) fusion partners, maltose binding protein (MBP), Protein A, glutathione S-transferase (GST), fluorescent protein sequences (*e*.*g*. GFP), epitope tags such as myc, FLAG and haemagglutinin tags.

The isolated peptides, variant and/or derivatives of the present disclosure may be produced by any means known in the art, including but not limited to, chemical synthesis and recombinant DNA technology.

Chemical synthesis is inclusive of solid phase and solution phase synthesis. Such methods are well known in the art, although reference is made to examples of chemical synthesis techniques as provided in Chapter 9 of SYNTHETIC VACCINES Ed. Nicholson (Blackwell Scientific Publications) and Chapter 15 of CURRENT PROTOCOLS IN PROTEIN SCIENCE Eds. Coligan et al., (John Wiley & Sons, Inc. NY USA 1995-2008). In this regard, reference is also made to International Publication WO 99/02550 and International Publication WO 97/45444.

Recombinant proteins may be conveniently prepared by a person skilled in the art using standard protocols as for example described in Sambrook et al., MOLECULAR CLONING. A Laboratory Manual (Cold Spring Harbor Press, 1989), in particular Sections 16 and 17; CURRENT PROTOCOLS IN MOLECULAR BIOLOGY Eds. Ausubel et al., (John Wiley & Sons, Inc. NY USA 1995-2008), in particular Chapters 10 and 16; and CURRENT PROTOCOLS IN PROTEIN SCIENCE Eds. Coligan et al., (John Wiley & Sons, Inc. NY USA 1995-2008), in particular Chapters 1, 5 and 6.

An aspect of the disclosure that does not belong to the invention provides a method of increasing modifying, altering, or changing a peptide with an amino acid sequence set forth in SEQ ID NO:1, SEQ ID NO:5, or SEQ ID NO:10, or an amino acid sequence at least 70% identical thereto, including the step of incorporating one or more amino acid insertions, deletions, or substitutions into the amino acid sequence of the peptide.

Preferably, modifying, altering, or changing the peptide increases or enhances one or more biological activities of the peptide. In preferred embodiments, the biological activity is selected from activity on cell proliferation, and activity on wound healing.

In certain preferred embodiments wherein the peptide that is modified according to this aspect is a peptide with the amino acid sequence set forth in SEQ ID NO:11, or a variant thereof, the method includes the step of inserting and/or deleting a valine residue into the amino acid sequence of the peptide. With reference to FIG. 2(A), the amino acid sequences set forth in SEQ ID NOS:2-5 feature a valine insertion relative to the amino acid sequence set forth in SEQ ID NO:11, between a CysI residue and a CysII residue of SEQ ID NO:11. Additionally, the amino acid sequences set forth in SEQ ID NOS:2-5 feature a valine deletion relative to the amino acid sequence set forth in SEQ ID NO: 11, between a CysIII residue and CysIV residue of SEQ ID NO:11. As set forth in FIGS. 4-5 peptides with an amino acid sequence set forth in SEQ ID NOS:2-5 demonstrated an increased or enhanced activity in respect of cell proliferation and/or wound healing, relative to a peptide with the amino acid sequence set forth in SEQ ID NO:11.

In certain preferred embodiments wherein the peptide that is modified according to this aspect is a peptide with the amino acid sequence set forth in SEQ ID NO:11, or a variant thereof, the method includes the step of substituting an alanine residue for a cysteine residue. Preferably, the alanine/cysteine substitution results in a cysteine cysteine motif in the amino acid sequence of the peptide. With reference to FIG. 2(A), the amino acid sequence set forth in SEQ ID NO:5 features a cysteine substitution relative to an alanine of SEQ ID NO:11. The cysteine substation is located between a CysIII residue and a CysIV residue of SEQ ID NO:11, and forms a cysteine cysteine motif in SEQ ID NO:5.

In certain preferred embodiments wherein the peptide that is modified is a peptide with the amino acid sequence set forth in SEQ ID NO:5, or a variant thereof, the method includes the step of substituting a proline residue for an alanine residue. With reference to Table 3, SEQ ID NOS:6-8 feature a alanine substitution relative to a proline of SEQ ID NO:5. As set forth FIG. 12, peptides with the amino acid sequence set forth in each of SEQ ID NOS:6-8 showed higher percentage increase cell proliferation rate, relative to a peptide with the amino acid sequence set forth in SEQ ID NO:5.

Another aspect of the disclosure provides an isolated peptide produced according to the method of the preceding aspect. Preferably, said isolated peptide has increased or enhanced biological activity.

The invention also provides an isolated nucleic acid encoding the isolated peptide of any one of SEQ ID NOS:1-10.

The term *"nucleic acid"* as used herein designates single- or double-stranded DNA and RNA. DNA includes genomic DNA and cDNA. RNA includes mRNA, RNA, RNAi, siRNA, cRNA and autocatalytic RNA. Nucleic acids may also be DNA-RNA hybrids. A nucleic acid comprises a nucleotide sequence which typically includes nucleotides that comprise an A, G, C, T or U base. However, nucleotide sequences may include other bases such as inosine, methylycytosine, methylinosine, methyladenosine and/or thiouridine, although without limitation thereto.

In one aspect, the isolated nucleic acid is in a genetic construct that comprises the isolated nucleic acid operably linked or connected to one or more other genetic components. A genetic construct may be suitable for therapeutic delivery of the isolated nucleic acid or for recombinant protein production in a host cell.

Broadly, the genetic construct is in the form of, or comprises genetic components of, a plasmid, bacteriophage, a cosmid, a yeast or bacterial artificial chromosome as are well understood in the art. Genetic constructs may be suitable for maintenance and propagation of the isolated nucleic acid in bacteria or other host cells, for manipulation by recombinant DNA technology and/or expression of the nucleic acid of the invention or an encoded protein.

For the purposes of host cell expression, the genetic construct is an expression construct. Suitably, the expression construct comprises the nucleic acid of the invention operably linked to one or more additional sequences in an expression vector. An *"expression vector"* may be either a self-replicating extra-chromosomal vector such as a plasmid, or a vector that integrates into a host genome.

By *"operably linked'* is meant that said additional nucleotide sequence(s) is/are positioned relative to the nucleic acid of the invention preferably to initiate, regulate or otherwise control transcription.

Regulatory nucleotide sequences will generally be appropriate for the host cell used for expression. Numerous types of appropriate expression vectors and suitable regulatory sequences are known in the art for a variety of host cells.

Typically, said one or more regulatory nucleotide sequences may include, but are not limited to, promoter sequences, leader or signal sequences, ribosomal binding sites, polyadenylatioin sequences, transcriptional start and termination sequences, translational start and termination sequences, and enhancer or activator sequences.

Constitutive, repressible or inducible promoters as known in the art are contemplated by the invention.

The expression construct may also include an additional nucleotide sequence encoding a fusion partner (typically provided by the expression vector) so that the recombinant protein is expressed as a fusion protein, as hereinbefore described.

The expression construct may also include an additional nucleotide sequence encoding a selection marker such as amp^{R}, neo^{R} or kan^{R}, although without limitation thereto.

In particular embodiments relating to delivery of isolated nucleic acids to a wound or to a subject, the expression construct may be in the form of plasmid DNA, suitably comprising a promoter operable in an animal cell (*e.g.* a CMV, an α A-crystallin or SV40 promoter). In other embodiments, the nucleic acid may be in the form of a viral construct such as an adenoviral, vaccinia, lentiviral or adeno-associated viral vector.

In a further aspect, the invention provides a host cell transformed with a nucleic acid molecule or a genetic construct described herein.

Suitable host cells for expression may be prokaryotic or eukaryotic. For example, suitable host cells may include but are not limited to mammalian cells (*e.g. HeLa, Cos, NIH-3T3, HEK293T, Jurkat cells),* yeast cells *(e.g. Saccharomyces cerevisiae),* insect cells *(e.g. Sf9, Trichoplusia ni)* utilized with or without a baculovirus expression system, plant cells (*e.g. Chlamydomonas reinhardtii, Phaeodactylum tricornutum*) or bacterial cells, such as *E*. *coli.* Introduction of genetic constructs into host cells (whether prokaryotic or eukaryotic) is well known in the art, as for example described in CURRENT PROTOCOLS IN MOLECULAR BIOLOGY Eds. Ausubel et al., (John Wiley & Sons, Inc. 1995-2015), in particular Chapters 9 and 16.

In particular aspects, the invention provides use of the isolated peptide disclosed herein, such as comprising the amino acid sequence of any one of SEQ ID NOS:1-10, for promoting in vitro cell proliferation, migration and/or wound healing. Also provided is use of a nucleic acid encoding an isolated peptide disclosed herein, or a genetic construct or vector comprising the same, for promoting in vitro cell proliferation, migration, and/or wound healing.

An aspect of the invention provides a method of promoting in vitro cell proliferation and/or migration, said method including the step of contacting one or more cells with the isolated peptide or an encoding nucleic acid to thereby initiate, stimulate or facilitate proliferation and/or migration of the one or more cells.

Another aspect of the disclosure, which does not belong to the claimed invention, provides a method of healing a wound, said method including the step of contacting the wound with the isolated peptide or an encoding nucleic acid to thereby at least partly heal the wound.

Thus the isolated peptide disclosed herein may promote the proliferation and/or migration of cells that facilitate wound healing in a subject. This may alternatively or additionally include promoting migration of cells to the wound that facilitate wound healing. Such cells may include macrophages, neutrophils, epidermal cells, keratinocytes, dendritic cells (e.g. Langerhans cells), vascular cells, fibroblasts, platelets, lymphocytes and/or progenitors of any of these cells, although without limitation thereto.

As generally used herein, a *"wound"* may be any damaging abrasion to, or physical breach of the integumentary system, such as the skin, and include cuts, lesions, ulcers and burns.

In certain embodiments, which do not belong to the claimed invention, the disclosure provides treatment of a wound, inclusive of abrasions, cuts, lesions, ulcers and burns. By *"treatment"* is meant a therapeutic course of action that at least partly ameliorates, reduces, removes or suppresses one or more symptoms or outcomes of a disease, disorder or condition. In some embodiments, the treatment may alternatively or additionally include prophylaxis by which recurrence or reappearance of the one or more symptoms or outcomes of the disease, disorder or condition is at least partly prevented.

In some embodiments, the step of contacting the wound with the isolated peptide or encoding nucleic acid may include systemic administration of the isolated peptide or encoding nucleic acid to the subject or topical administration of the isolated peptide or encoding nucleic acid to the wound.

In some embodiments, for the purposes of administration to the subject, the isolated peptide, or encoding nucleic acid, may be in the form of a pharmaceutical composition comprising the isolated peptide or encoding nucleic acid together with a pharmaceutically acceptable carrier, diluent or excipient.

By *"carrier, diluent or excipient"* is generally meant a solid or liquid filler, diluent, solvent, vehicle or encapsulating substance that may be safely used in systemic administration. Depending upon the particular route of administration, a variety of carriers, well known in the art may be used. These carriers may be selected from a group including sugars, starches, cellulose and its derivatives, malt, gelatine, talc, calcium sulfate, glidants, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates and pyrogen-free water.

A useful general reference describing carriers, diluents and excipients is Remington's Pharmaceutical Sciences (Mack Publishing Co. N.J. USA, 1991).

Any suitable procedure is contemplated for producing compositions, such as vaccine compositions. Exemplary procedures include, for example, those described in New Generation Vaccines (1997, Levine et al., Marcel Dekker, Inc. New York, Basel, Hong Kong)

Any safe route of administration may be employed for providing an animal with the composition of the invention. For example, topical, oral, rectal, parenteral, sublingual, buccal, intravenous, intranasal, intra-articular, intra-muscular, intradermal, subcutaneous, inhalational, intraocular, intraperitoneal, intracerebroventricular and transdermal administration may be employed.

Dosage forms include tablets, dispersions, suspensions, salves, ointments, creams, pastes, dispersions, injections, solutions, syrups, troches, capsules, nasal sprays, suppositories, aerosols, transdermal patches and the like. These dosage forms may also include injecting or implanting controlled releasing devices designed specifically for this purpose or other forms of implants modified to act additionally in this fashion. Controlled release of the therapeutic agent may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, the controlled release may be effected by using other polymer matrices, liposomes and/or microspheres.

Compositions of suitable for administration may be presented as discrete units such as capsules, caplets, sachets, functional foods/feeds or tablets, or as a powder or granules or as a solution or a suspension in an aqueous liquid, a non-aqueous liquid, an oil-in-water emulsion or a water-in-oil liquid emulsion.

The composition may be administered in a manner compatible with the dosage formulation, and in such amount as is immunologically effective. The dose administered to a subject should be sufficient to effect a beneficial response in a subject over an appropriate period of time. The quantity of agent(s) to be administered may depend on the subject to be treated inclusive of the age, sex, weight and general health condition thereof, factors that will depend on the judgement of the practitioner.

As generally used herein, a *"subject"* may be any animal inclusive of mammals, preferably a human. Veterinary applications may be suitable for wound healing in animals such as fish, poultry, domestic pets, racehorses, livestock and other non-human subjects, although without limitation thereto.

In still yet another further aspect, the disclosure provides a method of producing an agent that promotes cell proliferation, migration and/or wound healing, said method including the step of identifying, engineering, screening or designing an analogue, mimetic or agonist of the isolated peptide disclosed herein that promotes cell proliferation, migration and/or wound healing.

It will be appreciated that the present invention provides insight into the folding of *Ov*-GRN-1 and that the N-terminal region contributes to bioactivity. Given the difficulties in producing *Ov*-GRN-1 in high quantities, the development of peptides derived from the protein that maintain or increase bioactivity (such as SEQ ID NOS:1-10) may facilitate identifying engineering, screening or designing an analogue, mimetic or agonist of the isolated peptide that could be a lead molecule for development of novel wound healing agents.

The agent may be a peptide or other protein, a small organic molecule, mono, oligo- or polysaccharide, lipid, nucleic acid or combination of these having a cell proliferation, migration and/or wound healing activity that at least partly mimics that of the isolated peptide disclosed herein. In some advantageous embodiments, the bioactivity of the agent may be greater than that of the isolated peptide when compared molecule to molecule.

In some embodiments, the agent may be rationally designed or engineered *de novo* based on desired or predicted structural characteristics or features that indicate the agent has a cell proliferation, migration and/or wound healing activity that at least partly mimics that of the isolated peptide disclosed herein. In other embodiments, the agent may be identified by screening a library of molecules without initial selection based on desired or predicted structural characteristics or features that indicate the agent has a cell proliferation, migration and/or wound healing activity that at least partly mimics that of the isolated peptide disclosed herein. Such libraries may comprise randomly generated or directed libraries of proteins, peptides, nucleic acids, phage display libraries, libraries of naturally-occurring molecules and/or combinatorial libraries of synthetic organic molecules.

Non-limiting examples of techniques applicable to the design and/or screening of candidate modulators may employ X-ray crystallography, NMR spectroscopy, computer assisted screening of structural databases, computer-assisted modelling or biochemical or biophysical techniques which detect, model or predict molecular folding and/or binding interactions, as are well known in the art.

Biophysical and biochemical techniques which identify molecular interactions include competitive radioligand binding assays, co-immunoprecipitation, fluorescence-based assays including fluorescence resonance energy transfer (FRET) binding assays, electrophysiology, analytical ultracentrifugation, label transfer, chemical cross-linking, mass spectroscopy, microcalorimetry, surface plasmon resonance and optical biosensor-based methods, such as provided in Chapter 20 of CURRENT PROTOCOLS IN PROTEIN SCIENCE Eds. Coligan et al., (John Wiley & Sons, 1997-2015) Biochemical techniques such as two-hybrid and phage display screening methods are provided in Chapter 19 of CURRENT PROTOCOLS IN PROTEIN SCIENCE Eds. Coligan et al., (John Wiley & Sons, 1997-2015).

It will be understood that an agent identified, engineered, screened or designed as disclosed herein may be suitable for use in methods of promoting cell proliferation, migration and/or wound healing as previously described.

Also provided is an antibody or antibody fragment that specifically binds the isolated peptide disclosed herein. By *"specifically binds"* is meant that the antibody or antibody fragment binds the isolated peptide with a substantially greater affinity than another protein, such as a wild-type granulin protein.

The antibody may be polyclonal or monoclonal as are well known in the art. Antibody fragments include single chain fragments such as scFv fragments, Fab and Fab'2 fragments, diabodies and triabodies, although without limitation thereto. Polyclonal antibodies may be produced by immunization with the isolated peptide followed by serum antibody purification. Monoclonal antibodies may be produced by immunization with the isolated peptide followed by spleen cell fusions and antibody purification, or may be produced by recombinant DNA technology. Recombinant antibody or antibody fragments may be engineered to comprise desired antigen-binding amino acid sequences (e.g CDR sequences) and/or modified to facilitate administration to a particular subject with reduced likelihood of elicting an unwanted immune responses to xenogeneic portions of the antibody (e.g humanized).

So that the invention may be fully understood and put into practical effect, reference is made to the following non-limiting examples.

### EXAMPLES

### Example 1. Development of a potent wound healing agent based on the granulin scaffold

### Materials and Methods

### Peptide synthesis and purification

Truncated granulin peptides were synthesised using manual solid-phase peptide synthesis using fluorenylmethyloxycarbonyl (FMOC) chemistry. Peptides were assembled on 2-chlorotrityl chloride resin (Auspep, Australia). Amino acids were activated using 2-(1H-benzotriazol-1-yl)-1,1,3,3-tetramethyluronium hexafluorophosphate (HBTU - Iris Germany) in peptide grade *dimethylformamide* (DMF -Auspep, Australia). Peptides were cleaved using a mixture of 95% TFA/2.5% TIPS/2.5% H₂O. The TFA was removed by evaporation with nitrogen and ice-cold diethyl ether was added to the residue. Ether was removed by filtration and the peptide was dissolved in 40% acetonitrile/water mixture containing 0.1% Trifluoroacetic acid (TFA) and subsequently freeze-dried. The resulting crude peptides were purified with reverse phase high performance liquid chromoatography (RP-HPLC) on a C-18 preparative column (Phenomenex Jupiter 10µm C₁₈ 300Å 250x21.2 mm). Gradients of 1%/min of 0%-80% solvent B (90% acetonitrile in 0.045% TFA in H₂O) and solvent A (aqueous 0.045% TFA in H₂O) were used and the eluent was monitored at 215 and 280 nm. Peptides were oxidised by stirring a solution of the peptide in 100 mM ammonium bicarbonate (pH 8.2) containing 5 mM reduced glutathione and left overnight at room temperature and purified using RP-HPLC on a C-18 preparative column (Phenomenex Jupiter 10µm C₁₈ 300Å 250x21.2 mm).

To confirm the disulfide connectivity of the truncated peptides, *Ov*-GRN₁₂₋₃₄ was synthesised with selective protection of the cysteine residues. Cys1 and Cys14 were side-chain protected with ACM groups and Cys8 and Cys23 with (Trt) protecting groups. Following cleavage and purification of the crude peptide the disulfide bond between Cys8 and Cys23 was formed in 100 mM ammonium bicarbonate and the peptide was purified using the procedure described above. The S-ACM groups were subsequently removed by stirring 2 mg of peptide in 0.5 mL TFA, 10 uL anisole and 25 mg silver trifluoromethanesulfonate at 4°C for 1.5 h. Cold ether (10 mL) was added to the mixture and the precipitate collected by centrifugation. The precipitate was washed twice with ether and oxidized, without further purification, overnight using a solution of 50% DMSO in 0.5 M HCl. The solution was diluted 15 times with water and the fully folded peptide was purified by HPLC using 1% ACN gradient on a C-18 preparative column (Phenomenex Jupiter 10µm C₁₈ 300Å 250x21.2 mm).

### Auto-induction of recombinant protein expression in E. coli

*Ov-grn-1* pET41a or *Escherichia coli* thioredoxin *(trx)* cDNAs contained within the pET32a (Novagen) plasmid were transfected into BL21 *E. coli* cells (Life Technologies) and used to create recombinant proteins with auto-induction as described^{4, 22}. Briefly, ZYM-5052 culture media were supplemented with 100 µM Fe(III)Cl₃ and 100 µg/L kanamycin to produce recombinant protein (r*Ov*-GRN-1) or 50 µg/L ampicillin to produce TRX. Two hundred (200) ml of inoculated media in a one-litre baffled Erlenmeyer flask was incubated overnight at 37°C at 300 rpm rotation to induce expression with auto-induction.

### Recombinant Protein Purification

Purification of r*Ov*-GRN-1 was achieved using an AKTA10 purification system at 4°C (GE Healthcare)²³. The BL21 *E. coli* pellet was lysed with 3 freeze/thaw cycles followed by sonication on ice with a Q4000 unit (Qsonix). Twenty (20) g of the resulting insoluble pellet was solubilized in 400 ml urea-containing nickel binding buffer (8 M urea/300 mM NaCl/50 mM imidazole/50 mM sodium phosphate pH 8 [Sigma]) at 4°C for 24 h with slow agitation. The 0.22 µM filtered supernatant was passed over 2 × 5 ml Histrap IMAC nickel columns (GE Healthcare) and washed with increasing imidazole concentrations (two column volumes [CV] at 50 mM/5 CV at 100 mM) and eluted with 500 mM imidazole in binding buffer. The control TRX protein was expressed in the same fashion but under native conditions (without chaotropic agents) and purified with Histrap IMAC Nickel columns²³.

### Protein refolding and purification

Refolding of urea-denatured r*Ov*-GRN-1 was performed with 28 mL of G10 Sephadex (GE) resin on a XK16/20 column (GE) as described²³. A 120 ml Superdex 30 XK16/60 column (GE) was used to fractionate 3 ml of refolded r*Ov*-GRN-1 into 150 mM NaCl, 50 mM sodium phosphate, pH 6, at a flow rate of 1 ml/min. Fractions containing r*Ov*-GRN-1 monomer eluting at a size equivalent of ~1 kDa (based on the fold of granulin proteins despite a denatured molecular size of 10.4 kDa) were pooled. Protein concentration was determined by a combination of microplate Bradford assay (Biorad) and absorbance at 280 nm.

### NMR spectroscopy and structure determination

Purified peptides were dissolved in 90%H₂O/10% D₂O to provide a ~0.2 mM stock. 2D ¹H-¹H TOCSY, ¹H-¹H NOESY, ¹H-¹H DQF-COSY, ¹H-¹⁵N HSQC, and ¹H-¹³C HSQC spectra were acquired at 290 K using a 600 MHz AVANCE III NMR spectrometer (Bruker, Karlsruhe, Germany) equipped with a cryogenically cooled probe. Spectra were recorded with an interscan delay of 1 s. NOESY spectra were acquired with a mixing time of 200 ms, and TOCSY spectra were acquired with an isotropic mixing period of 80 ms. All spectra were assigned using CCPNMR²⁴ based on the approach described in Wuthrich et al.²⁵ The αH secondary shifts were determined by subtracting the random coil ¹H NMR chemical shifts of Wishart et al.²⁶ from experimental αH chemical shifts.

The three-dimensional structures of *Ov*-GRN₁₂₋₃₄ and *Ov*-GRN_{12-35_3s} were determined. The 2D NOESY spectra were automatically assigned and an ensemble of structures calculated using the program CYANA²⁷. Torsion-angle restraints predicted using TALOS+ were used in the structure calculations. Disulfide-bond connectivities (Cys1-Cys14, Cys8-Cys23) were included in the calculations for *Ov*-GRN₁₂₋₃₄ because these bonds were confirmed by selective protection of the cysteine residues. Selective protection of the cysteine residues was not used for *Ov*-GRN_{12-35_3s} in an attempt to isolate the most energetically favourable form. Consequently, the structures were calculated with the 15 possible disulfide connectivities. An analysis of the CYANA target functions was carried out to determine the most likely connectivity. Structures were visualised using MOLMOL²⁸.

### Mammalian cell culture

The non-malignant cholangiocyte cell line H69 is a SV40-transformed human bile duct epithelial cell line derived from human liver, kindly provided by Dr. Gregory J. Gores, Mayo Clinic, Rochester, Minnesota. H69 cells^{23, 29, 30} were maintained in T75cm² vented flasks (Corning) as monolayers as described³¹ with minor modifications. Cells were maintained with regular splitting using 0.25% trypsin (Life Technologies) every 2-5 days in complete media [RPMI (Sigma) with growth factor-supplemented specialist complete media³⁰ [DMEM/F12 with high glucose, 10% FCS, 1×antibiotic/antimycotic, 25 µg/ml adenine, 5 µg/ml insulin, 1 µg/ml epinephrine, 8.3 µg/ml holo-transferrin, 0.62 µg/ml, hydrocortisone, 13.6 ng/ml T3 and 10 ng/ml EGF - Life Technologies]. Low nutrient media for cell proliferation assays was 5% complete media, i.e. 0.5% FCS and 1/20^{th} of the growth factor concentrations listed above for complete media. The identities (human-derived) of the cell line were confirmed with single tandem repeat (STR) analysis in January 2015 (15/15 positive loci across 2 alleles) and mycoplasma free at the DNA Diagnostics Centre (DDC)-medical (U.S.A.), accredited/certified by CAP, ISO/IEC 17025:2005 through ACLASS.

### Cell proliferation monitoring in real time using xCELLigence

Cells were seeded at 1,500 cells/well in 180 µl complete media (above) in E-plates (ACEA Biosciences) and grown overnight while monitored with an xCELLigence SP system (ACEA Biosciences) which monitors cellular events in real time by measuring electrical impedance across interdigitated gold micro-electrodes integrated into the base of tissue culture plates³². Cells were washed three times with PBS prior to addition of 180 µl of low nutrient media (above) and incubated for a minimum of 6 h before further treatment. Treatments were prepared at 10× concentration and added to each well in a total volume of 20 µl. The xCELLigence system recorded cell indexes at intervals of one hour for 5-6 days following treatment. Readings for the cell index were normalized prior to treatment and cell proliferation ratios were determined from biological quadruplicates and represent the relative numbers of cells compared to control cells at day 4. Comparisons of induction of cell proliferation in response to treatments were accomplished using two-way ANOVA test with Dunnett's multiple comparison correction, using GraphPad Prism 6.02.

### Mouse wounding assay

These studies were conducted with the approval of the James Cook University Small Animal Ethics Committee, applications A1806 and A2204, as described⁶. Briefly, 4-5 female BALB/c mice per group *were* anesthetized (intraperitoneal xylazine 16 mg/kg; ketamine 80 mg/kg), after which a skin-deep wound on the crown of the head was inflicted using a 5 mm biopsy punch (Zivic instruments). Betadine liquid antiseptic (Sanofi) was applied followed by application of 50 µl that contained either 71 pmoles of Regranex (treatment of 71 pmoles equals 1 µg per 0.25 cm² wound, as recommended by manufacturer Smith and Nephew), 56 pmoles of r*Ov*-GRN-1, Ov-GRN-1 peptides, control peptide (EADRKYDEVARKLAMVEADL), TRX or PBS suspended in 1.5% methylcellulose (Sigma). Wounds were photographed daily and after blinding treatment groups the area of the lesion was measured with ImageJ software and plotted as percent of wound closure from original wound images. Rates of wound rates were compared with two-way ANOVA test with Dunnett's correction for multiple comparisons, using GraphPad prism 6.02. Each mouse wounding study was conducted at least twice to provide reproducibility.

### Results

### Design and synthesis of truncated Ov-GRN-1 peptides

To determine if the N-terminal region of *Ov*-GRN-1 can fold independently several truncated peptides were designed and synthesized using FMOC chemistry. The sequences of the synthetic peptides are shown in Figure 2A.

*Ov*-GRN₁₋₃₅, *Ov*-GRN₈₋₃₈ and *Ov*-GRN₁₂₋₃₄ all contain four cysteine residues equivalent to Cys I, Cys II, Cys III and Cys V in the full length protein (for the remainder of the report, Roman numerals refer to the numbering present in the full length protein). Cys IV and Cys VI were predicted to form disulfide bonds with Cys VII and Cys IX respectively, based on the three-dimensional structure of carp granulin-1⁷. In the truncated analogues Cys IV and Cys VI were replaced with alanine residues to prevent disulfide bond formation between these residues. Selective protection of the cysteine residues was used to direct the folding to form the predicted disulfide connectivity (i.e. Cys I-Cys III and Cys II-Cys V).

*Ov*-GRN-1 contains an extended N-terminal tail (11 residues prior to the first cysteine residue) not present in the majority of granulins, and these residues were included in *Ov*-GRN₁₋₃₅ to determine if they play a role in the bioactivity. The N-terminus was truncated and the C-terminus extended in *Ov*-GRN₈₋₃₈ to provide an analogue with a similar number of residues to the carp granulin-1 truncated peptide. ***Ov*-GRN₁₂₋₃₄** is the minimal sequence that contains the four cysteine residues (CysI, CysII, CysIII and CysV) and was designed to determine if the N- and C-terminal regions are required for folding and activity.

An additional peptide was synthesised (*Ov*-GRN_{12-35_3s}) with a truncated N-terminus but containing the first six cysteines of *Ov*-GRN-1 (the "3s" refers to the presence of three disulfide bonds in the peptide). This peptide is analogous to mammalian paragranulin (above) in terms of the cysteine residues. It was synthesized without selective protection of the cysteine residues and the major conformation was purified for analysis of its structure and activity.

### Structural analysis with NMR spectroscopy

NMR spectroscopy was employed to analyse the structure of the peptides. The one-dimensional spectra of *Ov*-GRN₁₋₃₅, *Ov*-GRN₈₋₃₈ and *Ov*-GRN₁₂₋₃₄ have limited dispersion in the amide regions consistent with a lack of β-sheet structures despite formation of the two native disulfide bonds. Two-dimensional spectra (TOCSY and NOESY) were used to assign the resonances, and the secondary shifts were determined by subtracting random coil shifts¹⁶ from the αH shifts. The secondary shifts are similar over the equivalent residues for these three peptides, as shown in Figure 2B, indicating that the structures were similar and consequently, that the differences in the N- and C-termini of these peptides did not influence the overall fold. Furthermore, the secondary shifts were consistent with a lack of β-sheet structure as they are primarily negative and β-sheet structures are characterised by positive secondary shifts. The three-dimensional structure of *Ov*-GRN₁₂₋₃₄ was determined using NMR spectroscopy, as shown in Figure 3A. In contrast to the characteristic granulin fold, the structure comprised turns and a region of 3₁₀ helix. The structure statistics are provided in Supplementary Table 2.

In contrast to the two disulfide bond-containing *Ov*-GRN-1 peptides, *Ov*-GRN_{12-35_3s}, with three disulfide bonds has more dispersion in the amide region in the one-dimensional NMR spectrum. Furthermore, additional peaks were present in the spectra, likely due to isomerisation of the proline residues. Despite these additional peaks, the major conformation was fully assigned, and the secondary shifts were similar to the truncated carp granulin-1¹⁴ (Figure 3B), which indicates the similarity of the overall structures. Truncated carp granulin-1, comprising residues 1-30, has previously been synthesised with Cys IV and Cys VI replaced with serine residues, and was shown to form a β-sheet structure¹⁴. Here we synthesised carp granulin₁₋₃₀ with Cys IV and Cys VI replaced with alanine residues to be consistent with the truncated peptides of *Ov*-GRN-1. Only minor variations were evident between the published¹⁴ chemical shifts of carp granulin-1 with the serine substitutions and the peptide with the alanine substitutions (Figure 6), indicating that the overall fold is still maintained.

To confirm if the structure of *Ov*-GRN_{12-35_3s} was similar to carp granulin₁₋₃₀, three-dimensional structures were calculated using CYANA. Structures were initially calculated without disulfide bond restraints. In these structures a β-hairpin was present from residues 14-23, but residues 1-8 were not defined. The lack of definition for residues 1-8 prevented an analysis of the sulfur-sulfur distances providing insight into the most likely connectivity. Therefore, an alternative approach was used whereby the structures were calculated with the 15 possible disulfide bond connectivities. This approach has previously been used for disulfide-rich peptides such as the cyclotides to analyse the disulfide bond connectivities^{17, 18}. The CYANA target functions for the 15 connectivities for *Ov*-GRN_{12-35_3s} are shown in Table 1. The connectivity with the lowest CYANA target function was CysI-CysIII, CysII-CysV and CysIV-CysVI. The three-dimensional structure of *Ov*-GRN_{12-35_3s} with this connectivity is shown in Figure 3A and the structure statistics provided in Supplementary Table S1. The most well defined region of the molecule was the β-hairpin between residues 14-23. The N-terminal region, encompassing CysI and CysII displayed marked structural disorder.

### Cell proliferation

The influence of the *Ov*-GRN-1 peptides on proliferation of H69 cholangiocytes in real time was assessed using xCELLigence technology and dose response curves were determined for the peptides. *Ov*-GRN_{12-35_3s} at a final concentration of 2 µM resulted in a 41% increase in cell growth compared to control peptide (p<0.0001) (Figure 4A). A dose response curve similar to that obtained for *Ov*-GRN-1 was observed with Ov-GRN_{12-35_3s} treatment, characterized by significantly increased cell proliferation at final concentrations of ≥15 nM (p<0.05). The two disulfide bonded *Ov*-GRN-1 peptides were less potent at nanomolar concentrations, but at 2 µM promoted significant cell proliferation (14-25% above peptide control; p<0.01) with dose response curves typified by *Ov*-GRN₁₂₋₃₄ (Figure 4A).

This is in contrast to carp granulin₁₋₃₀ that induced minimal cell proliferation (non-significant) at all concentrations tested, and maximum proliferation of 9% over peptide controls at 32 nM. The response at 400 nM of all the Ov-GRN peptides (Figure 4B) highlights the enhanced potency of the three disulfide bonded peptide (*Ov*-GRN_{12-35_3s}) compared to the two disulphide bonded peptides. *Ov*-GRN_{12-35_3s} promoted a highly significant (p<0.0001) increase in cell proliferation (26% over peptide controls) compared to the remaining peptides that induced minimal proliferation, of which the most potent was *Ov*-GRN₁₋₃₅ (9% non-significant increase over peptide control).

### Mouse wound healing model

The truncated *Ov*-GRN-1 peptides formulated with methylcellulose were tested in a mouse model of wound healing. All *Ov*-GRN-1 peptides exhibited potent activity (Figure 5A, B) when applied topically compared to control peptide in methycellulose. The *Ov*-GRN-1 peptides, *Ov*-GRN-1 protein and Regranex significantly improved healing compared to peptide control on days 2-4 (p<0.05). As wounds closed, differences among treatments waned and significant differences were unapparent beyond day 4. Regranex and the various granulin peptides showed near identical bestfit curves and intact *Ov*-GRN-1 was the only compound tested here that provided significant improvement over Regranex on days 3 and 4 (p<0.05; Figure 5B). Significant differences were not observed between the various negative control groups formulated with methylcellulose, including PBS vehicle control, peptide control, and thioredoxin (TRX) recombinant protein control.

When healing at day 4 was evaluated relative to PBS vehicle from each biological replicate, treatment of wounds with *Ov*-GRN-1 protein and peptides significantly accelerated wound healing compared to controls (p<0.01 at day 4: 26-41% over PBS). Although the *Ov*-GRN-1 protein, *Ov*-GRN₁₋₃₅ and *Ov*-GRN₁₂₋₃₄ (37-41% over PBS) provided improved healing compared to Regranex (29% over PBS), none of these comparisons reached significance at the day 4 time point.

### Discussion

Elucidating the structure/activity relationships of granulins has been challenging given the sequence and structural variations in this protein family. Regions with bioactivity are poorly understood, and uncertainty remains about potential receptors for this growth factor^{19, 20}.

*Ov*-GRN-1 appears to have distinct folding pathways compared to other granulins. The N-terminal region of *Ov*-GRN-1, comprising two native disulfide bonds (CysI-CysIII and CysII-V), does not fold independently into a native-like β-hairpin structure, in contrast to carp granulin-1 and human granulin A. It is noteworthy that the introduction of a third, non-native disulfide bond in *Ov*-GRN_{12-35_3s} results in a β-hairpin structure similar to that present in the carp granulin-1 and human granulin A peptides^{14, 15}. The disulfide bond connectivity of *Ov*-GRN_{12-35_3s} appears to comprise the two native disulfide bonds (CysI-CysIII and Cys II-V) in addition to the CysIV-CysVI disulfide bond. If the bond pairs are conserved across species^{7, 9}, the latter bond is predicted not to be present in the full length *Ov*-GRN-1, as CysIV is predicted to bond to CysVII and CysVI to CysIX.

The paragranulin (half-granulin) domain of mammal progranulin contains the equivalent six cysteine residues present in *Ov*-GRN_{12-35_3s} and is biologically active²¹, which suggests that *Ov*-GRN_{12-35_3s} potentially contains the same disulfide connectivity. Carp granulin₁₋₃₀ peptide might accommodate this CysI-CysIII, Cys II-CysV, CysIV-CysVI connectivity¹⁴. Although carp granulin₁₋₃₀ peptide contains only the two native disulfide bonds (CysI-CysIII and Cys II-CysV), analysis of the structure indicates that the side-chains of the serine residues, which replace CysIV and CysVI, are in close proximity, and suggest that it is feasible for these cysteine residues to form a disulfide bond.

The disulfide connectivity in *Ov*-GRN_{12-35_3s} has implications for the structure of full-length *Ov*-GRN-1, which has not been experimentally determined because sufficient quantities of correctly folded recombinant material remain unavailable. Therefore, the disulfide connectivity of the native protein has not been shown to conform to the connectivity originally shown for carp granulin-1⁷. It is conceivable that the protein contains a disulfide domain comprising the first six cysteine residues (equivalent to that seen in *Ov*-GRN_{12-35_3s}), and a second domain containing the last six cysteine residues. Without the structure of the full-length protein and a comparison to the native protein secreted by the parasite, this remains speculation. However, previous reports revealed ambiguity in the disulfide connectivity of granulins^{9, 15}. The structures of human granulin A and F have well-defined N-terminal regions, but disordered C-terminal regions prevented characterisation of all the disulfide bonds. Furthermore, chemical analysis of the disulfide connectivity of human granulin A was inconclusive⁹.

In addition to providing insight into the folding of *Ov*-GRN-1, the current study revealed that the N-terminal region contributes to the bioactivity and the β-hairpin of *Ov*-GRN_{12-35_3s} further enhanced cell proliferation activity. However, the β-hairpin structure is far from the complete story in regard to proliferative activity, as the carp granulin₁₋₃₀ peptide contains dual β-hairpins and in contrast to the *Ov*-GRN-1 peptides, showed no substantial proliferation at the eight concentrations tested (10 nM - 2 µM). A comparison of the sequences of carp granulin-1 with *Ov*-GRN-1 reveals that there are only two conserved non-cysteine residues between CysI and CysVI. This lack of conservation in the loop sequences likely accounts for the differences in both folding and bioactivity.

Despite the lack of native structure, the two disulfide bond containing *Ov*-GRN-1 peptides promoted cell proliferation at high concentrations (>800 nM) and stimulated significant healing of cutaneous wounds in mice. *Ov*-GRN_{12-35_3s} was the most potent peptide in the cell proliferation assay, but was no more active *in vivo* than the other *Ov*-GRN-1 peptides. If the β-hairpin of *Ov*-GRN_{12-35_3s} is involved in wound healing *in vivo* we did not observe a difference in mice. Cell proliferation activity may be cell line-specific, or alternatively the concentrations tested in mouse wound repair were not optimal. In either case, the activity observed in mice may be of greater biological and therapeutic consequence than findings from the *in vitro* analysis. In the future, we envision exploring a range of cells from diverse organs and tissues and investigation of mice that exhibit deficits in wound healing in order to increase our understanding of the role of *Ov*-GRN-1 structure-activity relationships.

To conclude, structural analysis with NMR spectroscopy suggested that *Ov*-GRN-1 exhibits unique folding properties compared with other granulins, presumably resulting from primary sequence. We have identified a bioactive region of Ov-GRN-1, which is likely to be less immunogenic and more readily produced than the full-length recombinant protein. Peptides and derivatives of liver fluke granulin that maintain the bioactivity represent a key advance towards identification of a novel therapies for treatment of wounds.

### Summary

During analysis of bioactive region(s) of Ov-GRN-1, a set of four truncated analogues were synthesized and characterized structurally using NMR spectroscopy. Peptides derived from the N-terminal region of *Ov*-GRN-1 comprising either two or three disulfide bonds drove proliferation of a human cholangiocyte cell line and displayed potent wound healing in mice. Peptides from Ov-GRN-1 that contain only two native disulfide bonds lack the β-hairpin structure characteristic of granulins. Remarkably, the introduction of a non-native disulfide bond was critical for formation of β-hairpin structure. Peptides derived from *Ov*-GRN-1 are superb leads for novel wound healing drugs as they are likely to be less immunogenic than the full-length protein and more convenient to produce.

### Example 2. Insights into the folding of a liver-fluke derived granulin

### Introduction

Granulins are a large family of disulfide-rich proteins with diverse biological functions including influencing cell growth³³. There is limited sequence conservation amongst the granulin domains but all contain twelve cysteine residues, with a conserved framework³⁴. The most well studied granulin in terms of structure is carp granulin-1, which display a stack of β-hairpins stapled together with the six disulfide bonds (Fig. 7-A)³⁵. Despite the conserved cysteine framework, the structure/function relationships are complex with some granulin domains displaying cell proliferative activity and some having inhibitory effects on cell growth^{36, 37}.

The liver-fluke granulin, *Ov*-GRN-1, isolated from *Opisthorchis viverrini,* displays potent wound healing activity *in vivo*³⁴*,* but low yields in the recombinant expression have limited its development as a therapeutic. Elucidating the structure/function relationships has also been impacted by the difficulties in producing significant quantifies for study. However, we have shown that truncated analogues of Ov-GRN-1 represent functional mimetics³⁴ with potential in treating chronic wounds where the normal tissue repair mechanisms are overwhelmed, such as diabetic ulcers³⁸⁻⁴¹.

Although our truncated analogues of *Ov*-GRN-1 have begun to provide insight into the folding and the bioactive region, questions remain regarding the important features for secondary structure formation and structural stability. For instance, the peptide (*Ov*-GRN_{12-35_3s}) corresponding to residues 12-35 in *Ov*-GRN-1, comprises only one β-hairpin in contrast to a truncated form of carp granulin-1³⁴. Intriguingly, this peptide contains a non-native disulfide bond based on the predicted connectivity, and the role of this bond in secondary structure stabilisation is not fully understood (Fig. 7-B). Ov-GRN_{12-35_3s} also displays evidence of additional conformations in the NMR spectra. These additional conformations are likely to be the result of proline *cis*/*trans* isomerisation but further study is required to determine which proline residues are involved. In the current study we have used mutational studies to determine the role of the proline residues in structure, folding and activity. Determining the most structurally stable and potent analogue is likely to facilitate the development of *Ov-*GRN-1 derived peptides as novel wound healing agents.

### Materials and Methods

### Peptide synthesis, purification and characterisation

Granulin analogues were synthesised by a stepwise solid phase peptide synthesis procedure on a Protein Technologies PS3 synthesiser. The Fmoc amino acid derivatives (Auspep, Australia) were activated using HCTU (Iris, Germany) and coupled on the 2-chlorotrityl chloride resin with DIPEA/DMF. The peptide was cleaved from the resin using the following cleavage cocktail: 95% TFA: 2.5% TIPS: 2.5% dH₂O. In the next step, peptide was precipitated with ice cold diethyl ether and dissolved in 50% Acetonitrile : 50% dH₂O (0.1%TFA v/v) and subsequently lyophilised. The resulting white powders were purified by reversed-phase HPLC using a C-18 preparative column (Phenomenex Jupiter 250x21.2 mm) and 1% gradient with acetonitrile-water mixtures containing 0.05% TFA (v/v). The eluents were monitored at 214 and 280 nm and the mass determined using MALDI TOF/TOF spectrometer.

### Disulfide formation

Disulfide bonds were formed by overnight air oxidation of 0. 1mg/ml peptide in 0.1M ammonium bicarbonate (pH 8-8.2) containing 5mM reduced glutathione at room temperature for 24 h; The solution was acidified, filtered and purified on a C-18 preparative column using RP-HPLC and the peptide mass was analysed using a 5800 MALDI TOF/TOF spectrometers.

### NMR spectroscopy and structure analysis

Samples were prepared from lyophilised peptide at a concentrations of about 0.2 mM in 90%H₂O : 10% D₂O. All NMR spectra were recorded on a 600 MHz AVANCE III NMR spectrometer (Bruker, Karlsruhe, Germany). 2D ¹H-¹H TOCSY, ¹H-¹H NOESY, ¹H-¹H DQF-COSY, ¹H-¹⁵N HSQC, and ¹H-¹³C HSQC at 290 K were used for assignment. All spectra were recorded with an interscan delay of 1 s. NOESY spectra were acquired with mixing times of200 ms, and TOCSY spectra were acquired with isotropic mixing periods of 80 ms. All spectra were assigned using CCPNMR⁴² based on the approach described in Wuthrich et al.⁴³. The αH secondary shifts were determined by subtracting the random coil ¹H NMR chemical shifts of Wishart et al.⁴⁴ from experimental αH chemical shifts. The 2D NOESY spectra were assigned and an ensemble of structures calculated using the program CYANA⁴⁵. A total of 100 initial structures were calculated using the CYANA program. Torsion-angle restraints predicted using TALOS N were used in the structure calculations. Structures were visualised using MOLMOL⁴⁶.

### Mammalian cell culture

H69 cell line, non-malignant cholangiocyte cell line was obtained from Dr. Gregory J. Gores, Mayo Clinic, Rochester, Minnesota³⁴. H69 cells were grown and maintained as previously described³⁴ in DMEM/F12 (Life Technologies) containing 1× antibiotic/antimycotic and 15 mM HEPES, supplemented with 10% fetal bovine serum (FBS) (Gibco, Scotland) at 37C and 5% incubator. Cell proliferation assays were performed with modified DMEM/F12 media supplemented with 0.5% FBS and particular hormone and growth factors in a certain concentration range as listed in the previous study³⁴.

### Cell proliferation monitoring in real time using xCELLigence

Proliferation study in real time was performed using xCELLigence SP system (ACEA Biosciences) as described previously³⁴. H69 cells were plated into a 96-well xCELLigence E-plate (ACEA Biosciences) at a density of2000 cells/well; then placed in the xCELLigence system positioned in a 5% CO₂ incubator at 37° C and monitored overnight⁴⁷. Then, the complete media was replaced with 180 µl of starvation media (modified DMEM/F12 media supplemented with 5% FBS as described previously³⁴) and incubated for at least 6 hour. Treatments were added to each well in a total volume of 20 µl to provide 200 nM final concentrations. Over 48h hour of measurement, cells reached confluence and the system records the cell index (CI). Cell proliferation rate was calculated as the relative numbers of treated cells compared to control cells over time (48 hours in culture). GraphPad Prism 6.02 was used for one-way ANOVA followed by Dunnett's multiple comparison test. Each treatment was measured in quadruplicate.

### Results

### Design and Synthesis of GRN_{12-35_3s} mutants

To determine which proline residues are involved in adopting multiple conformations, three single mutants (P2A, P4A, P10A) of GRN_{12-35_3s} in which each mutant has one of the three prolines changed to alanine were chemically synthesised. All three proline residues were replaced with alanine in an additional analogue termed GRN_{3Ala}. The sequences of the synthetic *Ov*-GRN-1 truncated peptides are given in Table 1.

All peptides were chemically synthesised using FMOC solid phase peptide synthesis. The crude peptides were purified using RP-HPLC and mass analysis carried out using MALDI mass spectrometry. The disulfide bonds were formed in 0.1 M ammonium bicarbonate and 5 mM glutathione at room temperature for 24 hours. The HPLC traces of the oxidative folding reactions are given in Fig. 8. For the majority of the peptides a relatively sharp, early eluting peak is present. The yield of this early eluting peak is significantly higher in the GRN_{3Ala} mutant compared to the other peptides. Under the conditions used in the current study, GRN_{12-35_3s} does not fold efficiently into a single isomer. For all peptides, with the exception of GRN_{P10A}, the major peaks (highlighted with an asterisk in Fig. 8) were isolated from each reaction for further characterisation.

The large number of peaks present in the folding reaction of GRN_{P10A}, even following a 48-hour oxidation period, prevented the purification of a single major disulfide isomer.

### Structural analysis with NMR spectroscopy

The structures of the purified or partially purified fractions for the GRN_{12-35_3s} analogues were analysed using NMR spectroscopy. The one-dimensional spectra of all peptides have significant dispersion in the amide region consistent with the presence of β-sheet structure. Analysis of the TOCSY and NOESY spectra for the individual proline mutants indicates the presence of multiple conformations most likely as a result of isomerisation of the proline residues. By contrast, the GRN_{3Ala} peptide did not appear to have multiple conformations (Fig. 9).

Two-dimensional spectra (TOCSY and NOESY) were used to assign the major conformations, and the secondary shifts were determined by subtracting random coil shifts⁴⁸ from the αH shifts. The secondary shifts are generally similar over the equivalent residues for all proline mutants compared to the N-terminal *Ov*-GRN-1 peptide, GRN_{12-35_3s}, as shown in Fig. 10, indicating that the overall structures are similar. The stretches of positive secondary shifts are consistent with the presence of β-sheet structure.

To confirm if the structures of the *Ov*-GRN-1 peptides maintain a fold similar to GRN_{12-35_3s}, three-dimensional structures of selected analogues were determined using the program CYANA. GRN_{3Ala} was chosen for full structural analysis because of the efficient folding, lack of conformational heterogeneity and to determine the influence of the proline residues on the overall fold. The disulfide bonding pattern of GRN_{12-35_3s} was previously predicted to be CysI-CysIII, CysII-CysV and CysIV-CysVI⁴⁹. The three-dimensional structure of GRN_{3Ala} shares the β-hairpin at the C-terminal region of the peptide with GRN_{12-35_3s}, but the N-terminal region contains an α-helix from residues 4 to 11 as shown in Fig. 11.

### Cell proliferation monitoring in real time using xCELLigence

In addition to structural studies, to gain insight into the structure-function relationships of *Ov*-GRN_{12-35_3s}, all engineered peptides were tested in an *in vitro* cell proliferation assay. Fig. 12 demonstrates the rates of cell proliferation for each peptide from xCELLigence plate readings over 48 hours. The individual proline mutants showed significant cell proliferation compared to the negative control peptide (20 residue peptide from tropomyosin). The proliferation rates of GRN24_{P2A}, _{P4A} and _{P10A} were 131.7% (P<0.0001), 141.6% (*P*<0.0001) and 131.4% (*P*<0.0001), respectively, relative to the control peptide. By contrast, GRN_{3Ala} did not have statistically significant effect (P = 0.90) on the cell proliferation compared to the control peptide.

### Discussion

*Ov*-GRN-1 has potential in the development of a novel wound-healing agent, but there has been limited information on the structure/function relationships. Here we show that all three prolines residues in *Ov*-GRN_{12-35_3s} have a significant role in both the structure and the function.

Mutation of the individual proline residues did not disrupt the overall fold of *Ov-*GRN_{12-35_3s}, but multiple conformations were still present in the NMR spectra. By contrast, when all three proline residues were mutated to alanine residues a single set of peaks corresponding to a single conformation were observed in the NMR spectra. These results indicate that all three proline residues are involved with *cis*/*trans* isomerisation.

*Cis*/*trans* isomerisation can be a rate-determining step in the folding of proteins⁴⁹, and appears to be influencing the folding of *Ov*-GPN_{12-35_3s}. Although mutation of proline 10 with an alanine residue did not improve the folding relative to *Ov*-GRN_{12-35_3s}, the folding yields of GRN_{P2A} and GRN_{P4A} were improved. Prevention of *cis*/*trans* isomerisation by removal of all three proline residues resulted in the highest folding yield (Fig. 8). These results indicate that the proline residues are generally detrimental to the folding under the current conditions.

The proline residues also have an effect on *in vitro* cell proliferation. It appears likely that the lack of activity of GRN_{3Ala} is related to the perturbation of the structure at the N-terminal region. Removal of the proline residues allows a helical structure to form, and this conformation might not enable interaction with a binding partner. Given that replacing individual proline residues with alanine residues still results in cell proliferation, it appears unlikely that direct interaction with the proline residues is involved in bioactivity. However, since all of the 3 proline residues are within the first 10 residues of *Ov*-GRN_{12-35_3s} it appears that this region, rather than the C-terminal hairpin is important in the bioactivity.

Previous studies on human granulin modules indicate that sequence can have a significant influence on folding yields, consistent with our current study. In mammals, granulins are expressed as progranulin, which contains seven-and-a-half granulin modules³³. The seven granulin modules in human progranulin were expressed as thioredoxin fusion proteins in *E. coli*⁵⁰. The expressed peptides were purified and cleaved by recombinant enterokinase to release the granulin modules. Analysis of the folding by HPLC analysis and NMR analysis indicates that granulin A, C and F display relatively well defined structures, at least for regions of the peptides. By contrast, granulins B and E did not display significant dispersion in the NMR spectra, and granulins D and G, were reported to have multiple signals in the NMR spectra for unique protons⁵⁰. Granulins B and F contain a proline residue immediately following the first cysteine in the sequence, consistent with proline 2 in *Ov*-GRN_{12-35_3s}. Granulin B does not have a predominant, sharp peak in the HPLC profile of the folding reaction, but granulin F does⁵⁰. Based on this comparison is does not appear that this proline residue has a significant influence on folding of the full-length human granulin modules.

### Summary

Overall, our results highlight the importance of the proline residues in structure and function of the *Ov*-GRN_{12-35_3s}. In addition, the data provide essential information for the design of a new generation of *Ov*-GRN-1-based potent analogues.

### Example 3. Assessment of mouse wound healing activity of peptides.

Day 4 wound healing were assessed using peptides as described herein (*Ov*-GRN-1, *Ov*-GRN₁₋₃₅, *Ov*-GRN₈₋₃₈, *Ov*-GRN₁₂₋₃₄, *Ov*-GRN_{12-35_3s}, GRN24-3ala, GRN24-P2A, GRN24-P4A, and GRN27sps). Outcomes are shown (FIG. 13(B)) relative to 56 pmoles peptide control from treatments with 56 pmoles of recombinant *Ov*-GRN-1, *Ov*-GRN-1 peptides, thioredoxin (TRX) protein controls and 71 pmoles Regranex in 1.5% methylcellulose gel applied daily in 50 µl volume from days 0-4 to a ~0.2 cm² wound arising from biopsy punch to the scalp between the ears. No significant differences (ns) between the unrelated peptide control, PBS, or TRX protein control were noted at any time point. Peptides *Ov*-GRN-1, *Ov*-GRN₁₋₃₅, *Ov*-GRN₈₋₃₈, *Ov-*GRN₁₂₋₃₄, *Ov*-GRN_{12-35_3s}, GRN24-3ala, and GRN24-3p4a showed significant increases in wound healing relative to controls.

All panels: mean healing rates of 2-6 biological replicates of groups of 4-5 animals plotted with SEM bars. Groups have been marginally shifted left or right to aid viewing. Repeated measure 2-way ANOVA test with Dunnett's correction for multiple comparisons compare each group against peptide control. Significance against peptide/protein control signified by ^{∗∗∗∗} = p<0.0001, ^{∗∗∗} = p<0.001, ^{∗∗} = p<0.01, ^{∗} = p<0.05, ns=not significant. Color of asterisk or hash represents the relevant group.

Throughout the specification the aim has been to describe the preferred embodiments of the invention without limiting the invention to any one embodiment or specific collection of features. It will therefore be appreciated by those of skill in the art that, in light of the instant disclosure, various modifications and changes can be made in the particular embodiments exemplified without departing from the scope of the present invention.

### REFERENCES

1. He, Z., Ong, C.H., Halper, J., and Bateman, A. (2003). Progranulin is a mediator of the wound response. Nat. Med. 9: 225-229.
2. Mulvenna, J., Sripa, B., Brindley, P.J., Gorman, J., Jones, M.K., Colgrave, M.L., et al. (2010). The secreted and surface proteomes of the adult stage of the carcinogenic human liver fluke Opisthorchis viverrini. Proteomics. 10: 1063-1078.
3. Laha, T., Pinlaor, P., Mulvenna, J., Sripa, B., Sripa, M., Smout, M.J., et al. (2007). Gene discovery for the carcinogenic human liver fluke, Opisthorchis viverrini. BMC Genomics. 8: 189.
4. Smout, M.J., Laha, T., Mulvenna, J., Sripa, B., Suttiprapa, S., Jones, A., et al. (2009). A granulin-like growth factor secreted by the carcinogenic liver fluke, Opisthorchis viverrini, promotes proliferation of host cells. PLoS Pathog. 5: e1000611.
5. Smout, M.J., Sripa, B., Laha, T., Mulvenna, J., Gasser, R.B., Young, N.D., et al. (2011). Infection with the carcinogenic human liver fluke, Opisthorchis viverrini. Mol. Biosyst. 7: 1367-1375.
6. Smout, M.J., Sotillo, J., Laha, T., Papatpremsiri, A., Rinaldi, G., Pimenta, R.N., et al. (2015). Carcinogenic Parasite Secretes Growth Factor That Accelerates Wound Healing and Potentially Promotes Neoplasia. PLoS Pathog. 11: e1005209.
7. Hrabal, R., Chen, Z., James, S., Bennett, H.P., and Ni, F. (1996). The hairpin stack fold, a novel protein architecture for a new family of protein growth factors. Nat. Struct. Biol. 3: 747-752.
8. Ong, C.H., and Bateman, A. (2003). Progranulin (granulin-epithelin precursor, PC-cell derived growth factor, acrogranin) in proliferation and tumorigenesis. Histol. Histopathol. 18: 1275-1288.
9. Tolkatchev, D., Malik, S., Vinogradova, A., Wang, P., Chen, Z., Xu, P., et al. (2008). Structure dissection of human progranulin identifies well-folded granulin/epithelin modules with unique functional activities. Protein Sci. 17: 711-724.
10. Alquezar, C., de la Encarnacion, A., Moreno, F., Lopez de Munain, A., and Martin-Requero, A. (2016). Progranulin deficiency induces overactivation of WNT5A expression via TNF-alpha/NF-kappaB pathway in peripheral cells from frontotemporal dementia-linked granulin mutation carriers. J. Psychiatry Neurosci. 41: 225-239.
11. Park, B., Buti, L., Lee, S., Matsuwaki, T., Spooner, E., Brinkmann, M.M., et al. (2011). Granulin is a soluble cofactor for toll-like receptor 9 signaling. Immunity. 34: 505-513.
12. Yeh, J.E., Kreimer, S., Walker, S.R., Emori, M.M., Krystal, H., Richardson, A., et al. (2015). Granulin, a novel STAT3-interacting protein, enhances STAT3 transcriptional function and correlates with poorer prognosis in breast cancer. Genes Cancer. 6: 153-168.
13. Yip, C.W., Cheung, P.F., Leung, I.C., Wong, N.C., Cheng, C.K., Fan, S.T., et al. (2014). Granulin-epithelin precursor interacts with heparan sulfate on liver cancer cells. Carcinogenesis. 35: 2485-2494.
14. Vranken, W.F., Chen, Z.G., Xu, P., James, S., Bennett, H.P., and Ni, F. (1999). A 30-residue fragment of the carp granulin-1 protein folds into a stack of two beta-hairpins similar to that found in the native protein. J. Pept. Res. 53: 590-597.
15. Tolkatchev, D., Ng, A., Vranken, W., and Ni, F. (2000). Design and solution structure of a well-folded stack of two beta-hairpins based on the amino-terminal fragment of human granulin A. Biochemistry. 39: 2878-2886.
16. Wishart, D.S., Bigam, C.G., Holm, A., Hodges, R.S., and Sykes, B.D. (1995). 1H, 13C and 15N random coil NMR chemical shifts of the common amino acids. I. Investigations of nearest-neighbor effects. J. Biomol. NMR. 5: 67-81.
17. Saether, O., Craik, D.J., Campbell, I.D., Sletten, K., Juul, J., and Norman, D.G. (1995). Elucidation of the primary and three-dimensional structure of the uterotonic polypeptide kalata B1. Biochemistry. 34: 4147-4158.
18. Daly, N.L., Koltay, A., Gustafson, K., R., Boyd, M.R., Casas-Finet, J.R., and Craik, D.J. (1999). Solution structure by NMR of circulin A: a macrocyclic knotted peptide having anti-HIV activity. J. Mol. Biol. 285: 333-345.
19. Chen, X., Chang, J., Deng, Q., Xu, J., Nguyen, T.A., Martens, L.H., et al. (2013). Progranulin does not bind tumor necrosis factor (TNF) receptors and is not a direct regulator of TNF-dependent signaling or bioactivity in immune or neuronal cells. J. Neurosci. 33: 9202-9213.
20. Etemadi, N., Webb, A., Bankovacki, A., Silke, J., and Nachbur, U. (2013). Progranulin does not inhibit TNF and lymphotoxin-alpha signalling through TNF receptor 1. Immunol. Cell Biol. 91: 661-664.
21. Rollinson, S., Young, K., Bennion-Callister, J., and Pickering-Brown, S.M. (2016). Identification of biological pathways regulated by PGRN and GRN peptide treatments using transcriptome analysis. Eur. J. Neurosci.
22. Studier, F.W. (2005). Protein production by auto-induction in high density shaking cultures. Protein Expr. Purif. 41: 207-234.
23. Smout, M.J., Mulvenna, J.P., Jones, M.K., and Loukas, A. (2011). Expression, refolding and purification of Ov-GRN-1, a granulin-like growth factor from the carcinogenic liver fluke, that causes proliferation of mammalian host cells. Protein Expr. Purif. 79: 263-270.
24. Vranken, W.F., Boucher, W., Stevens, T.J., Fogh, R.H., Pajon, A., Llinas, M., et al. (2005). The CCPN data model for NMR spectroscopy: development of a software pipeline. Proteins. 59: 687-696.
25. Wüthrich, K. (1986). NMR of Proteins and Nucleic Acids, Wiley-Interscience, New York.
26. Wishart, D.S., Bigam, C.G., Yao, J., Abildgaard, F., Dyson, H.J., Oldfield, E., et al. (1995). 1H, 13C and 15N chemical shift referencing in biomolecular NMR. J. Biomol. NMR. 6: 135-140.
27. Guntert, P. (2004). Automated NMR structure calculation with CYANA. Methods Mol. Biol. 278: 353-378.
28. Koradi, R., Billeter, M., and Wüthrich, K. (1996). MOLMOL: a program for display and analysis of macromolecular structures. J. Mol. Graph. 14: 29-32.
29. Grubman, S.A., Perrone, R.D., Lee, D.W., Murray, S.L., Rogers, L.C., Wolkoff, L.I., et al. (1994). Regulation of intracellular pH by immortalized human intrahepatic biliary epithelial cell lines. Am. J. Physiol. 266: G1060-1070.
30. Matsumura, T., Takesue, M., Westerman, K.A., Okitsu, T., Sakaguchi, M., Fukazawa, T., et al. (2004). Establishment of an immortalized human-liver endothelial cell line with SV40T and hTERT. Transplantation. 77: 1357-1365.
31. Papatpremsiri, A., Smout, M.J., Loukas, A., Brindley, P.J., Sripa, B., and Laha, T. (2015). Suppression of Ov-grn-1 encoding granulin of Opisthorchis viverrini inhibits proliferation of biliary epithelial cells. Exp. Parasitol. 148: 17-23.
32. Xing, J.Z., Zhu, L., Jackson, J.A., Gabos, S., Sun, X.J., Wang, X.B., et al. (2005). Dynamic monitoring of cytotoxicity on microelectronic sensors. Chem. Res. Toxicol. 18: 154-161.
33. He, Z., et al., Progranulin is a mediator of the wound response. Nature medicine, 2003. 9(2): p. 225-229.
34. Bansal, P.S., et al., Development of a potent wound healing agent based on the liver fluke granulin structural fold. Journal of Medicinal Chemistry, 2017. 60(10): p. 4258-4266.
35. Hrabal, R., et al., The hairpin stack fold, a novel protein architecture for a new family of protein growth factors. Nature structural biology, 1996. 3(9): p. 747-52.
36. Plowman, G.D., et al., The epithelin precursor encodes two proteins with opposing activities on epithelial cell growth. Journal of Biological Chemistry, 1992. 267(18): p. 13073-13078.
37. Brown, C.A. and J. Halper, Mitogenic effects of transforming growth factor type e on epithelial and fibroblastic cells-comparison with other growth factors. Experimental cell research, 1990. 190(2): p. 233-242.
38. He, Z., et al., Progranulin is a mediator of the wound response. Nat Med, 2003. 9(2): p. 225-9.
39. Smout, M.J., et al., Infection with the carcinogenic human liver fluke, Opisthorchis viverrini. Molecular bioSystems, 2011. 7(5): p. 1367-1375.
40. Laha, T., et al., Gene discovery for the carcinogenic human liver fluke, Opisthorchis viverrini. BMC genomics, 2007. 8(1): p. 1.
41. Smout, M.J., et al., A granulin-like growth factor secreted by the carcinogenic liver fluke, Opisthorchis viverrini, promotes proliferation of host cells. PLoS Pathog, 2009. 5(10): p. e1000611.
42. Vranken, W.F., et al., The CCPN data model for NMR spectroscopy: development of a software pipeline. Proteins, 2005. 59(4): p. 687-96.
43. Wüthrich, K., NMR of Proteins and Nucleic Acids. 1986, New York: Wiley-Interscience. 292.
44. Wishart, D.S., et al., 1H, 13C and 15N chemical shift referencing in biomolecular NMR. J. Biomol. NMR, 1995. 6(2): p. 135-40.
45. Güntert, P., Automated NMR structure calculation with CYANA, in Protein NMR Techniques, A.K. Downing, Editor. 2004, Humana Press: Totowa, NJ. p. 353-378.
46. Koradi, R., M. Billeter, and K. Wuthrich, MOLMOL: a program for display and analysis of macromolecular structures. J. Mol. Graph., 1996. 14(1): p. 51-5, 29-32.
47. Xing, J.Z., et al., Dynamic monitoring of cytotoxicity on microelectronic sensors. Chem Res Toxicol, 2005. 18(2): p. 154-61.
48. Wishart, D.S., et al., 1H, 13C and 15N random coil NMR chemical shifts of the common amino acids. I. Investigations of nearest-neighbor effects. J. Biomol. NMR, 1995. 5: p. 67-81.
49. Reimer, U., et al., Side-chain effects on peptidyl-prolyl cis/trans isomerisation. Journal of molecular biology, 1998. 279(2): p. 449-460.
50. Tolkatchev, D., et al., Structure dissection of human progranulin identifies well-folded granulin/epithelin modules with unique functional activities. Protein Science, 2008. 17(4): p. 711-724.

### TABLES

**Table 1. CYANA target functions for the 15 possible disulfide bond connectivities present in Ov-GRN_{12-35_3s}.**

| Set Number | Disulfide bonds connectivity | Average Target Function ± SD |
|---|---|---|
| 1 | 1-14, 8-23,15-24 | 0.03 ± 0.003^{∗} |
| 2 | 1-14, 8-24, 15-23 | 5.94 ± 0.44 |
| 3 | 1-14, 8-15, 23-24 | 3.56 ± 0.090 |
| 4 | 1-8, 14-23, 15-24 | 3.73 ± 0.17 |
| 5 | 1-8, 14-15, 23-24 | 14.16 ± 0.23 |
| 6 | 1-8, 14-24, 15-23 | 5.68 ± 0.04 |
| 7 | 1-15, 8-14, 23-24 | 3.74 ± 0.36 |
| 8 | 1-15, 8-23, 14-24 | 1.88 ± 1.46 |
| 9 | 1-15, 8-24, 14-23 | 1.12 ± 0.16 |
| 10 | 1-23, 8-14, 15-24 | 0.09 ± 0.11 ^{∗} |
| 11 | 1-23, 8-15, 14-24 | 0.83 ± 0.19 |
| 12 | 1-23, 8-24, 14-15 | 9.46 ± 0.2 |
| 13 | 1-24, 8-14, 15-23 | 6.79 ± 0.2 |
| 14 | 1-24, 8-15, 14-23 | 1.3 ± 0.1 |
| 15 | 1-24, 8-23, 14-15 | 9.23 ± 018 |

| | | |
|---|---|---|
| ^{∗}The two connectivities with the lowest target functions are highlighted with an asterisk. The connectivity corresponding to Set Number 1 has the lowest target function indicating that the distance and angle restraints satisfy this connectivity better than the other connectivities, and it is therefore the most likely connectivity present in Ov-GRN_{12-35_3s}. | | |

**Table 2. Structural statistics for the Ov-GRN peptides ensemble**

| | | Ov-GRN₁₂₋₃₄ | Ov-GRN_{12-35_3s} |
|---|---|---|---|
| **Experimental restraints** | | | |
| Interproton distance restraints | | 252 | 113 |
| | Intraresidue | 78 | 46 |
| | Sequential | 139 | 52 |
| | Medium range (i-j<5) | 21 | 4 |
| | Long range (i-j≥5) | 14 | 44 |
| Disulfide-bond restraints | | 6 | 9 |
| Dihedral-angle restraints | | 14 | 10 |
| | | | |

| **R.m.s. deviations from mean coordinate structure (Å)** | | | |
|---|---|---|---|
| Backbone atoms | | 0.32 ± 0.15 | 0.65 ± 0.2^{∗} |
| All heavy atoms | | 0.93 ± 0.27 | 1.65 ± 0.33^{∗} |
| | | | |

| **Ramachandran Statistics** | | | |
|---|---|---|---|
| % in most favoured region | | 71.4 | 78.2 |
| % in additionally allowed region | | 28.6 | 21.8 |

| | | | |
|---|---|---|---|
| ^{∗} RMSD for residues 12-24. | | | |

**Table 3 Sequence of GRN_{12-35_3s} and its designed analogues. All Ov-GRN-1 truncated peptides contain six cysteine residues equivalent to Cys I, Cys II, Cys III, Cys IV and Cys V in the full-length protein. GRN(12-35_3s) = SEQ ID NO:5; GRN(P2A) = SEQ ID NO:6; GRN(P4A) = SEQ ID NO:7; GRN(P10A) = SEQ ID NO:8; GRN(3Ala) = SEQ ID NO:9.**

| Peptide | Sequence |
|---|---|
| GRN_{12-35_3s} | CPDPVYTCRPGQTCCRGLHGYGCC |
| GRN_{P2A} | CADPVYTCRPGQTCCRGLHGYGCC |
| GRN_{P4A} | CPDAVYTCRPGQTCCRGLHGYGCC |
| GRN_{P10A} | CPDPVYTCRAGQTCCRGLHGYGCC |
| GRN_{3Ala} | CADAVYTCRAGQTCCRGLHGYGCC |

## Claims

1. An isolated peptide consisting of the amino acid sequence:
¹Xₙ C ²XD ³XVYTCR ⁴XGQTC C/A RGLHGYGC ⁵Xₘ (SEQ ID NO:1),
wherein ¹Xₙ is a sequence of amino acids of length n, with n = 0-11 and ¹X being any amino acid;
wherein each of ²X, ³X, and ⁴X is P or A;
wherein ⁵Xₘ is a sequence of amino acids of length m, with m = 0-4 and ⁵X being any amino acid; and
wherein the isolated peptide comprises, or is capable of forming, two or three intrachain disulphide bonds.

2. The isolated peptide of Claim 1, wherein n = 3 and ¹Xₙ = SPS; or n = 4 and ¹Xₙ = RSPS; or n = 11 and ¹Xₙ = MDTLQPIRSPS.

3. The isolated peptide of claim 1 or 2, wherein m = 1, ⁵Xₘ = C or A; or m = 4 and ⁵Xₘ = APMD or CPMD.

4. The isolated peptide of any preceding claim consisting of the amino acid sequence set forth in any one of SEQ ID NOS: 2-10.

5. An isolated nucleic acid encoding the isolated peptide of any one of Claims 1-4.

6. A genetic construct comprising the isolated nucleic acid of Claim 5.

7. A host cell comprising the genetic construct of Claim 6.

8. A pharmaceutical composition comprising the isolated peptide of any one of Claims 1-4, or the isolated nucleic acid of Claim 5, together with a pharmaceutically acceptable carrier, diluent or excipient.

9. An isolated polypeptide of any one of claims 1-4 for use as a medicament.

10. The isolated nucleic acid of claim 5 for use as a medicament.

11. An isolated polypeptide of any one of claims 1-4 for use in the treatment of wounds.

12. The isolated nucleic acid of claim 5 for use in the treatment of wounds.

13. Use of the isolated polypeptide of any one of claims 1-4, or the isolated nucleic acid of claim 5, or the genetic construct of claim 6, or the pharmaceutical composition of claim 8, for initiating, promoting, stimulating, or facilitating in vitro cell proliferation and/or in vitro cell migration.

14. A method of promoting in vitro cell proliferation and/or migration, said method including the step of contacting one or more cells in vitro with the isolated peptide of any one of Claims 1-4, or the isolated nucleic acid of Claim 5, to thereby initiate, stimulate or facilitate proliferation and/or migration of the one or more cells in vitro.

## Patentansprüche

1. Isoliertes Peptid bestehend aus der Aminosäuresequenz:
¹XₙC ²XD ³XVYTCR ⁴XGQTC C/A RGLHGYGC ⁵Xₘ (SEQ ID NO:1),
wobei ¹Xₙ eine Sequenz von Aminosäuren einer Länge n ist, wobei n = 0-11 und ¹X irgendeine Aminosäure ist;
wobei jedes von ²X, ³X und ⁴X P oder A ist;
wobei ⁵Xₘ eine Sequenz von Aminosäuren einer Länge m ist, wobei m = 0-4 und ⁵X irgendeine Aminosäure ist; und
wobei das isolierte Peptid zwei oder drei Disulfidbindungen umfasst oder fähig ist, diese auszubilden.

2. Isoliertes Peptid nach Anspruch 1, wobei n = 3 und ¹Xₙ = SPS; oder n = 4 und ¹Xₙ = RSPS; oder n = 11 und ¹Xₙ = MDTLQPIRSPS.

3. Isoliertes Peptid nach Anspruch 1 oder 2, wobei m = 1, ⁵Xₘ = C oder A; oder m = 4 und ⁵Xₘ = APMD oder CPMD.

4. Isoliertes Peptid nach einem der vorstehenden Ansprüche, bestehend aus der Aminosäuresequenz, die in einer der SEQ ID NO: 2-10 dargelegt ist.

5. Isolierte Nukleinsäure, codierend für das isolierte Peptid nach einem der Ansprüche 1 bis 4.

6. Genetisches Konstrukt, umfassend die isolierte Nukleinsäure nach Anspruch 5.

7. Wirtszelle, umfassend das genetische Konstrukt nach Anspruch 6.

8. Pharmazeutische Zusammensetzung, umfassend das isolierte Peptid nach einem der Ansprüche 1 bis 4 oder die isolierte Nukleinsäure nach Anspruch 5, zusammen mit einem pharmazeutisch verträglichen Träger, Verdünner oder Hilfsstoff.

9. Isoliertes Polypeptid nach einem der Ansprüche 1 bis 4 zur Verwendung als ein Arzneimittel.

10. Isolierte Nukleinsäure nach Anspruch 5 zur Verwendung als ein Arzneimittel.

11. Isoliertes Polypeptid nach einem der Ansprüche 1 bis 4 zur Verwendung bei der Behandlung von Wunden.

12. Isolierte Nukleinsäure nach Anspruch 5 zur Verwendung bei der Behandlung von Wunden.

13. Verwendung des isolierten Polypeptids nach einem der Ansprüche 1 bis 4 oder der isolierten Nukleinsäure nach Anspruch 5 oder des genetischen Konstrukts nach Anspruch 6 oder der pharmazeutischen Zusammensetzung nach Anspruch 8 zum Einleiten, Fördern, Stimulieren oder Erleichtern einer In-vitro-Zellproliferation und/oder In-vitro-Zellmigration.

14. Verfahren zum Fördern der In-vitro-Zellproliferation und/oder-migration, wobei das genannte Verfahren den Schritt eines In Kontakt Bringen einer oder mehrerer Zellen in vitro mit dem isolierten Peptid nach einem der Ansprüche 1 bis 4 oder der isolierten Nukleinsäure nach Anspruch 5 einschließt, um dadurch die Proliferation und/oder Migration der einen oder der mehreren Zellen in vitro einzuleiten, zu stimulieren oder zu erleichtern.

## Revendications

1. Peptide isolé constitué de la séquence d'acides aminés :
¹Xₙ C ²XD ³XVYTCR ⁴XGQTC C/A RGLHGYGC ⁵Xₘ (SEQ ID NO : 1),
dans lequel ¹Xₙ est une séquence d'acides aminés de longueur n, avec n = 0 à 11 et ¹X étant n'importe quel acide aminé ;
dans lequel chacun de ²X, ³X, et ⁴X est P ou A ;
dans lequel ⁵Xₘ est une séquence d'acides aminés de longueur m, avec m = 0 à 4 et ⁵X étant n'importe quel acide aminé ; et
dans lequel le peptide isolé comprend, ou est capable de former, deux ou trois liaisons disulfure intrachaîne.

2. Peptide isolé selon la revendication 1, dans lequel n = 3 et ¹Xₙ = SPS ; ou n = 4 et ¹Xₙ = RSPS ; ou n = 11 et ¹Xₙ = MDTLQPIRSPS.

3. Peptide isolé selon la revendication 1 ou 2, dans lequel m = 1, ⁵Xₘ = C ou A ; ou m = 4 et ⁵Xₘ = APMD ou CPMD.

4. Peptide isolé selon une quelconque revendication précédente constitué de la séquence d'acides aminés indiquée dans l'une quelconque des SEQ ID NO : 2 à 10.

5. Acide nucléique isolé codant pour le peptide isolé selon l'une quelconque des revendications 1 à 4.

6. Construction génétique comprenant l'acide nucléique isolé selon la revendication 5.

7. Cellule hôte comprenant la construction génétique selon la revendication 6.

8. Composition pharmaceutique comprenant le peptide isolé selon l'une quelconque des revendications 1 à 4, ou l'acide nucléique isolé selon la revendication 5, conjointement avec un support, diluant ou excipient pharmaceutiquement acceptable.

9. Polypeptide isolé selon l'une quelconque des revendications 1 à 4 pour utilisation comme médicament.

10. Acide nucléique isolé selon la revendication 5 pour utilisation comme médicament.

11. Polypeptide isolé selon l'une quelconque des revendications 1 à 4 destiné à être utilisé dans le traitement de plaies.

12. Acide nucléique isolé selon la revendication 5 destiné à être utilisé dans le traitement de plaies.

13. Utilisation du polypeptide isolé selon l'une quelconque des revendications 1 à 4, ou de l'acide nucléique isolé selon la revendication 5, ou de la construction génétique selon la revendication 6, ou de la composition pharmaceutique selon la revendication 8, pour initier, promouvoir, stimuler ou faciliter la prolifération cellulaire in vitro et/ou la migration cellulaire in vitro.

14. Procédé de promotion de la prolifération et/ou de la migration cellulaire in vitro, ledit procédé comprenant l'étape de mise en contact d'une ou plusieurs cellules in vitro avec le peptide isolé selon l'une quelconque des revendications 1 à 4, ou l'acide nucléique isolé selon la revendication 5, pour de ce fait initier, stimuler ou faciliter la prolifération et/ou la migration de la ou des cellules in vitro.
